(19) Europäisches Patentamt / European Patent Office / Office européen des brevets

(11) **EP 3 378 384 A1**

(12) **EUROPEAN PATENT APPLICATION**
published in accordance with Art. 153(4) EPC

(43) Date of publication:
**26.09.2018  Bulletin 2018/39**

(21) Application number: **15908835.0**

(22) Date of filing: **20.11.2015**

(51) Int Cl.:
*A61B 5/02* (2006.01)

(86) International application number:
**PCT/JP2015/082809**

(87) International publication number:
**WO 2017/085896 (26.05.2017 Gazette 2017/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Fujitsu Limited**
**Kawasaki-shi, Kanagawa 211-8588 (JP)**

(72) Inventors:
• **MORI, Tatsuya**
  **Kawasaki-shi**
  **Kanagawa 211-8588 (JP)**
• **UCHIDA, Daisuke**
  **Kawasaki-shi**
  **Kanagawa 211-8588 (JP)**
• **MAEDA, Kazuho**
  **Kawasaki-shi**
  **Kanagawa 211-8588 (JP)**
• **INOMATA, Akihiro**
  **Kawasaki-shi**
  **Kanagawa 211-8588 (JP)**

(74) Representative: **Haseltine Lake LLP**
**Lincoln House, 5th Floor**
**300 High Holborn**
**London WC1V 7JH (GB)**

(54) **INFORMATION PROCESSING DEVICE, INFORMATION PROCESSING METHOD, AND INFORMATION PROCESSING PROGRAM**

(57)    An information processing apparatus (100) acquires pulse wave waveforms of each part of a subject obtained by analyzing images of each of the parts of a plurality of parts of the subject included in each photographed image of a plurality of photographed images obtained by photographing the subject. The information processing apparatus (100) calculates a first index value indicating a matching degree between at least any of the pulse wave waveforms among the acquired pulse wave waveforms of each of the parts of the subject. The information processing apparatus (100) calculates a second index value indicating a matching degree between partial waveforms of the pulse wave waveforms at least at any part of the subject among the acquired pulse wave waveforms of each of the parts of the subject. The information processing apparatus (100) determines whether or not the pulse wave waveforms at any part of the subject indicate an arrhythmia based on the calculated first index value and the calculated second index value.

FIG. 1

EP 3 378 384 A1

**Description**

CROSS-REFERENCE TO RELATED APPLICATION

This application is a continuation application of International Application PCT/JP2015/082809, filed on November 20, 2015 and designated the U.S., the entire contents of which are incorporated herein by reference.

Technical Field

The present invention relates to an information processing apparatus, an information processing method, and an information processing program.

Background Art

In the related art, there is a technology of measuring a pulse wave waveform of a subject by detecting a change in volume of blood that flows through a blood vessel of the subject based on a moving image obtained by photographing the subject. As a related art, for example, there is a technology of determining body movement of a person to be measured based on a characteristic value of a distribution waveform illustrating distribution of a light receiving level of a light receiving element in one direction by nipping a plurality of light receiving elements which receive transmitted light that is incident on the inside of the finger from a light generating element and has passed the finger with the finger. In addition, for example, there is a technology of processing data acquired from an input signal having physiological information that at least partially represents a periodic vital signal. In addition, for example, there is also a method of remotely monitoring vital signs by detecting a luminance signal in an image of a subject photographed by a video camera, such as a web camera.

Citation List

Patent Literature

PTL 1: Japanese Laid-open Patent Publication No. 2000-107147
PTL 2: Japanese Laid-open Patent Publication No. 2014-502187
PTL 3: Japanese Laid-open Patent Publication No. 2014-527863

Summary of Invention

Technical Problem

However, in the above-described related art, there is a case where it is not possible to analyze the pulse wave waveform of the subject with high accuracy. For example, in a case where the pulse wave waveform of the subject is disturbed, it is difficult to determine whether the pulse wave waveform is disturbed by noise caused by the body movement or the like of the subject, or the pulse wave waveform is disturbed by an arrhythmia or the like of the subject.
In one aspect, an object of the present invention is to provide an information processing apparatus, an information processing method, and an information processing program which can improve analysis accuracy related to an arrhythmia of the pulse wave waveform of the subject.

Solution to Problem

According to an aspect of the invention, there are provided an information processing apparatus, an information processing method, and an information processing program for acquiring pulse wave waveforms of each of a plurality of parts obtained by analyzing images of each of the parts of a subject included in each photographed image obtained by photographing the subject; calculating a first index value indicating a matching degree between the pulse wave waveforms of the acquired pulse wave waveforms of each of the parts; calculating a second index value indicating a matching degree between partial waveforms of the acquired pulse wave waveforms of each of the parts; determining whether or not the acquired pulse wave waveform indicates an arrhythmia based on a combination of the calculated first index value and the calculated second index value; and outputting a determination result as to whether or not to indicate the arrhythmia.

Advantageous Effects of Invention

According to one aspect of the present invention, it is possible to improve the analysis accuracy of the arrhythmia of the pulse wave waveform of the subject.

The object and advantages of the invention will be realized and attained by means of the elements and combinations particularly pointed out in the claims.

It is to be understood that both the foregoing general description and the following detailed description are exemplary and explanatory and are not restrictive of the invention, as claimed.

Brief Description of Drawings

[FIG. 1] FIG. 1 is an explanatory view illustrating an example of an information processing method according to an embodiment.

[FIG. 2] FIG. 2 is a view illustrating an example of a hardware configuration of an information processing apparatus 100.

[FIG. 3] FIG. 3 is a block diagram illustrating an example of a functional configuration of the information processing apparatus 100.

[FIG. 4] FIG. 4 is an explanatory view illustrating a first example of extracting images of each part of a subject.

[FIG. 5] FIG. 5 is an explanatory view illustrating a second example of extracting images of each of the parts of the subject.

[FIG. 6] FIG. 6 is an explanatory view illustrating a third example of extracting images of each of the parts of the subject.

[FIG. 7] FIG. 7 is an explanatory view (part 1) illustrating an example of generating pulse wave waveforms of each of the parts of the subject.

[FIG. 8] FIG. 8 is an explanatory view (part 2) illustrating an example of generating the pulse wave waveforms of each of the parts of the subject.

[FIG. 9] FIG. 9 is an explanatory view illustrating an example of setting an attention period.

[FIG. 10] FIG. 10 is an explanatory view illustrating a first example of calculating a first index value.

[FIG. 11] FIG. 11 is an explanatory view illustrating a second example of calculating the first index value.

[FIG. 12] FIG. 12 is an explanatory view illustrating an example of calculating a second index value.

[FIG. 13] FIG. 13 is an explanatory view illustrating an example of determining whether or not a partial waveform within an attention period indicates an arrhythmia according to a first determination pattern.

[FIG. 14] FIG. 14 is an explanatory view illustrating an example of determining whether or not the pulse wave waveform within a predetermined period indicates the arrhythmia according to the first determination pattern.

[FIG. 15] FIG. 15 is an explanatory view illustrating an example of determining whether or not the pulse wave waveform within the predetermined period indicates the arrhythmia according to a second determination pattern.

[FIG. 16] FIG. 16 is an explanatory view illustrating an example of determining whether or not a partial waveform within the attention period indicates the arrhythmia according to the second determination pattern.

[FIG. 17] FIG. 17 is an explanatory view illustrating an example of generating a model for specifying an arrhythmia region 1601.

[FIG. 18] FIG. 18 is a flowchart illustrating an example of an overall processing procedure.

[FIG. 19] FIG. 19 is a flowchart illustrating an example of a generation processing procedure.

[FIG. 20] FIG. 20 is a flowchart illustrating a first example of the overall processing procedure.

[FIG. 21] FIG. 21 is a flowchart illustrating a second example of the overall processing procedure.

[FIG. 22] FIG. 22 is a flowchart illustrating a third example of the overall processing procedure.

[FIG. 23] FIG. 23 is a flowchart illustrating a fourth example of the overall processing procedure.

[FIG. 24] FIG. 24 is a flowchart illustrating a fifth example of the overall processing procedure.

[FIG. 25] FIG. 25 is a flowchart illustrating a sixth example of the overall processing procedure.

Description of Embodiments

Hereinafter, embodiments of an information processing apparatus, an information processing method, and an information processing program according to the present invention will be described in detail with reference to the drawings.

(One Example of Information Processing Method According to Embodiment)

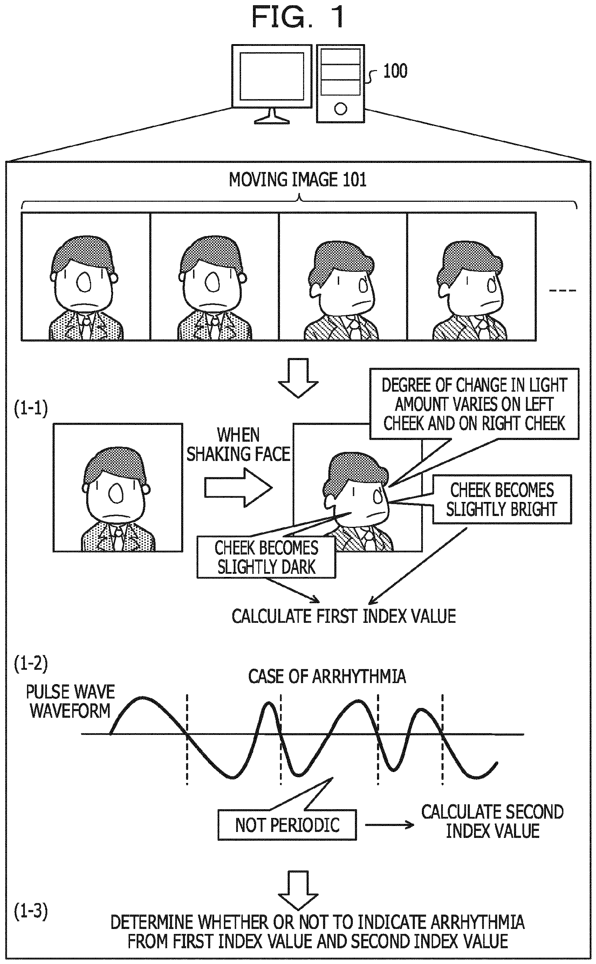

FIG. 1 is an explanatory view illustrating an example of the information processing method according to an embodiment. In FIG. 1, an information processing apparatus 100 is a computer that supports analysis of a pulse wave waveform of a subject. The subject is the object to measure the pulse wave waveform. The subject is, for example, a human being. The subject may be an animal. The pulse wave waveform is a waveform representing a temporal change in volume or pressure of blood that flows through a blood vessel of the subject with the beat of the heart.

Meanwhile, there has been a request to efficiently make determination concerning the health of the subject using biological information of the subject due to aging or an increase in medical expenses. For example, various types of information obtained from pulse wave waveforms in biological information of the subject are one of important indicators for viewing the life reaction of the subject, and are used for determining the health of the subject. Specifically, the pulse rate can be an index representing the health condition of the subject. In addition, in a case where a state where the pulse rate is abnormally high (so-called tachycardia) is generated or lasts frequently despite the absence of a psychological stress load, there is no doubt that the function of the heart normally works. In addition, the fluctuation of the pulse wave interval for each beat can be an index representing the stress applied to the subject. Further, the pulse wave waveform can be an index indicating the blood vessel age of the subject. Further, the pulse wave waveform can be an index indicating whether or not the subject is in a state of arrhythmia leading to serious diseases, such as heart failure or cerebral infarction. Arrhythmia is that the rhythm of heart rate and pulse is not regular. In the arrhythmia state, the pulse wave waveform becomes a waveform that is not periodic.

In this manner, much information on the health condition of the subject can be obtained from the pulse wave waveform. Therefore, it is desirable to measure the pulse wave waveform of the subject efficiently, on a day-to-day basis continuously and without consciousness of the subject by the simplest possible method. For example, it is desirable to measure the pulse wave waveform of the subject non-invasively and noncontactly, even when the subject does not operate any device. Here, for example, a technology for noninvasively and noncontactly measuring the pulse wave waveform of the subject that corresponds to the volume change of the blood that flows through the blood vessel by utilizing the fact that the amount of light absorbed by the blood vessel of the subject depends on the blood flow rate and by analyzing the luminance change of the body surface of the subject from the moving image of the subject, is considered.

Meanwhile, there is a case where noise is included in the measured pulse wave waveform. For example, in a case where there is body movement of the subject, a moving image obtained by photographing the subject tends to include a component of noise caused by the body movement of the subject in addition to the component that corresponds to the signal of the pulse wave waveform. Therefore, for example, by keeping the subject static, there is a case where the component of noise included in the pulse wave waveform of the subject is reduced and the measurement accuracy of the pulse wave waveform of the subject is improved.

However, in this case, it takes a burden to keep the subject static for a long time, and it is difficult to measure the pulse wave waveform of the subject without routine and continuous consciousness of the subject. Furthermore, when the subject may not keep static, the noise that appears in the pulse wave waveform becomes large due to the body movement and the like of the subject, and it is not possible to avoid the inclusion of noise in the pulse wave waveform. As a result, since the pulse wave waveform contains noise, there is a case where it is not possible to analyze the pulse wave waveform with high accuracy. For example, in a case where disturbance occurs in the pulse wave waveform due to noise, there is a case where it is erroneously determined that the pulse wave waveform is disturbed due to the arrhythmia or the like of the subject. The disturbance of the pulse wave waveform means that the pulse wave waveform becomes a waveform which is not periodic.

Here, in the present embodiment, the information processing method for improving analysis accuracy of the pulse wave waveform by calculating a first index value indicating likelihood of pulse waves of the pulse wave waveform of the subject and a second index value indicating how periodically the pulse wave waveform is periodic from the moving image of the subject, will be described.

The first index value is a value indicating whether or not the pulse wave waveform is disturbed by noise caused by the body movement or the like of the subject in a case where the pulse wave waveform of the subject is disturbed. The first index value indicates the degree of influence of the body movement of the subject on the pulse wave waveform. For example, the first index value is a value that indicates a matching degree between the pulse wave waveforms of the pulse wave waveforms of each of the parts obtained by detecting a change in volume of the blood that flows through the blood vessel at each of the parts of the subject. Each of the parts is, for example, a left cheek and a right cheek of the subject. The body movement of the subject is, for example, shaking of the head of the subject. The pulse wave waveform of the part is a waveform similar to a pulse wave generated based on the luminance change of the part surface.

Here, the value indicating the matching degree between the pulse wave waveforms indicates a state where the body movement of the subject becomes large as the value becomes small and the pulse wave waveform is easily disturbed by the noise caused by the body movement of the subject, and indicates that the likelihood of pulse waves is small. Specifically, in a case where the body movement of the subject is relatively large, the degree of change in light amount that corresponds to each of the parts varies, and the degree of change in color of each of the parts varies, and thus, the degree of change in pulse wave waveforms of each of the parts also varies, and the value indicating the matching degree becomes smaller. Meanwhile, in a case where the body movement of the subject is relatively small, since the difference in degree of change in color of each of the parts is relatively small, the value indicating the matching degree becomes large.

The second index value is a value that indicates how periodically the pulse wave waveform of the subject is periodic (degree of periodicity) and indicates how similar partial waveforms appear repeatedly in the pulse wave waveform of the subject. In addition, the second index value indicates how disturbed the pulse wave waveform of the subject is. For example, the second index value is a value that indicates a matching degree between the partial waveforms of the pulse wave waveforms of each of the parts obtained by detecting a change in volume of the blood that flows through the blood vessel at any part of the subject. Any parts is, for example, a face of the subject. Specifically, the second index value may be a value indicating the magnitude of the variation in the pulse wave interval of the pulse wave waveforms at any part of the subject.

Here, the value indicating the matching degree between the partial waveforms indicates that the disturbance of the pulse wave waveforms at any part of the subject becomes large as the value becomes small. Specifically, according to the body movement or the arrhythmia of the subject, the amplitude of the pulse wave waveforms at any part of the subject changes, partial waveforms similar to each other in the pulse wave waveforms at any part are not repeated, and the value indicating the matching degree between the partial waveforms becomes small. Meanwhile, when there is no body movement or arrhythmia of the subject, the pulse wave waveforms at any part becomes a periodic waveform that corresponds to the contraction or expansion of the heart, partial waveforms similar to each other in the pulse wave waveforms at any part are repeated, and the value indicating the matching degree between the partial waveforms become large.

The second index value may be, for example, a standard deviation of a difference between maximum values or minimum values adjacent to each other in the pulse wave waveforms at any part of the subject. In addition, the second index value may be, for example, the standard deviation of the pulse wave interval in the pulse wave waveforms at any part of the subject. Further, the second index value may be, for example, a peak width of spectral distribution obtained from the pulse wave waveforms at any part of the subject. Further, the second index value may be, for example, a peak ratio of the spectral distribution obtained from the pulse wave waveforms at any part of the subject. The peak ratio is, for example, a ratio between the maximum value and the second largest value in the spectral distribution.

In the example of FIG. 1, (1-1) the information processing apparatus 100 acquires the pulse wave waveforms of each of the parts obtained by analyzing the image of each of the parts of the plurality of parts of the subject included in each of the photographed images of the plurality of photographed images obtained by photographing the subject. The photographed image is data obtained by converting an optical signal from the subject at a certain point of time into an electric signal by a photoelectric conversion element. The photographed image is data expressed by the size of each color of RGB with respect to the color of the subject by, for example, a Red, Green, and Blue (RGB) color model.

The plurality of photographed images are a plurality of images arranged in chronological order in the order of photographing. The plurality of photographed images are, for example, a plurality of images obtained from a moving image 101. The moving image 101 is a series of frame images along the time series. The moving image 101 may have already been photographed or may be being acquired in real time. The plurality of photographed images are, for example, a series of frame images included in the moving image 101. The part is a part of the body of the subject or a predetermined part of the part of the body of the subject. The plurality of parts are, for example, a face, a left cheek and a right cheek.

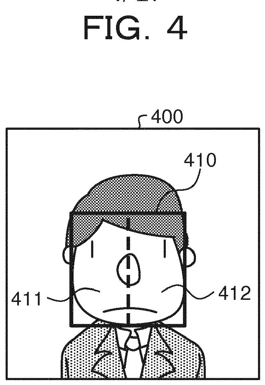

The information processing apparatus 100 obtains, for example, the pulse wave waveforms of each of the parts acquired by analyzing images of each of the parts of the face, the left cheek, and the right cheek of the subject included in the moving image 101 obtained by photographing the face of the subject, from another computer. The details of acquiring the pulse wave waveforms of each of the parts will be described later with reference to FIGs. 4 to 8, for example.

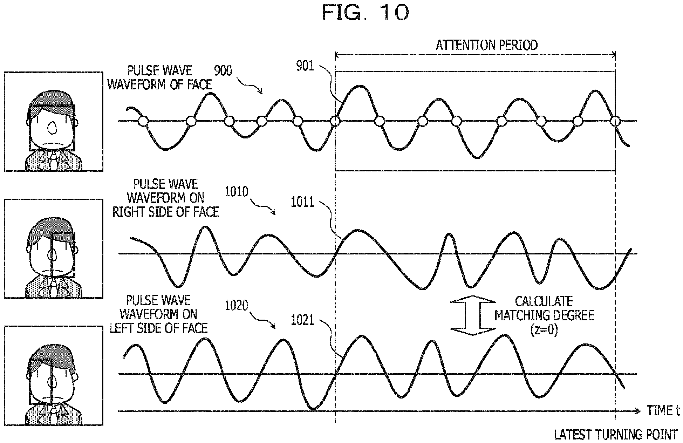

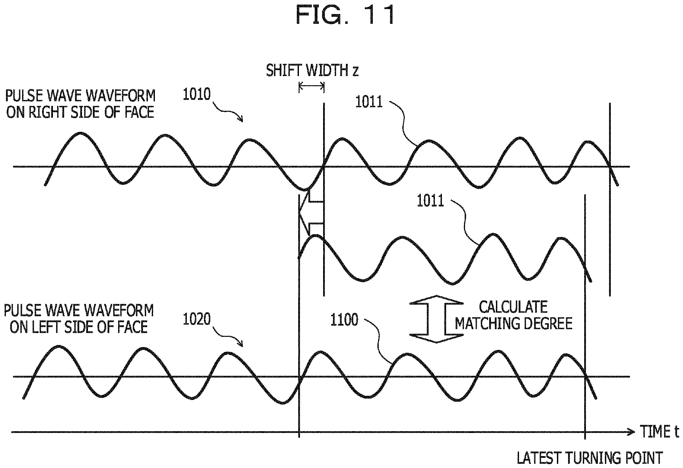

The information processing apparatus 100 calculates the first index value indicating the matching degree between at least one of the acquired pulse wave waveforms of each of the parts. The information processing apparatus 100, for example, calculates the first index value indicating the matching degree between the pulse wave waveforms of the left cheek and the right cheek among the acquired pulse wave waveforms of each of the parts, by using the correlation coefficient. The details of calculating the first index value will be described later with reference to FIGs. 10 and 11, for example.

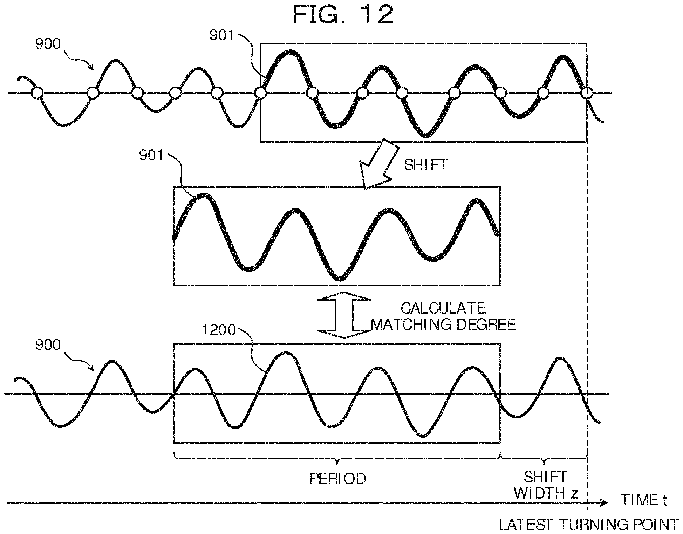

(1-2) The information processing apparatus 100 calculates a second index value indicating the matching degree between partial waveforms of the pulse wave waveforms of at least one of the parts, in the acquired pulse wave waveforms at each of the parts. The information processing apparatus 100, for example, calculates the second index value indicating the matching degree between the partial waveforms of the acquired pulse wave waveform of the face, by using the correlation coefficient. The information processing apparatus 100 may calculate the second index value from the acquired pulse wave waveform of the left cheek or the pulse wave waveform of the right cheek as long as the pulse wave waveform of the face has not been acquired. The details of calculating the second index value will be described later with reference to FIG. 12, for example.

(1-3) The information processing apparatus 100 determines whether or not the pulse wave waveforms at any part indicates an arrhythmia, based on the calculated first index value and the calculated second index value. The expression that the pulse wave waveform indicates an arrhythmia means that at least a part of the pulse wave waveform includes a waveform when the subject has an arrhythmia. The information processing apparatus 100 determines whether or not the pulse wave waveform of the face of the subject indicates an arrhythmia, for example, by at least one of a first determination pattern or a second determination pattern which will be described later. The information processing apparatus 100 may not be able to perform either one of the first determination pattern and the second determination pattern.

<One Example of Determining whether or not Arrhythmia is indicated by First Determination Pattern>

For example, the information processing apparatus 100 determines whether or not noise is included in the acquired pulse wave waveforms at any part based on the calculated first index value. Specifically, the information processing apparatus 100 determines that noise is included in the pulse wave waveform of the face in a case where the calculated first index value is less than a first threshold value, and determines that noise is not included in the pulse wave waveform of the face in a case where the calculated first index value is equal to or greater than the first threshold value. The fact that the noise is not included in the pulse wave waveform means that the component of noise included in the pulse wave waveform is small enough to be negligible in the analysis of the pulse wave waveform.

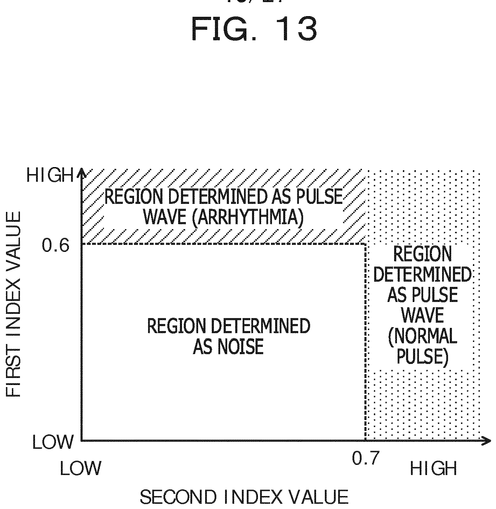

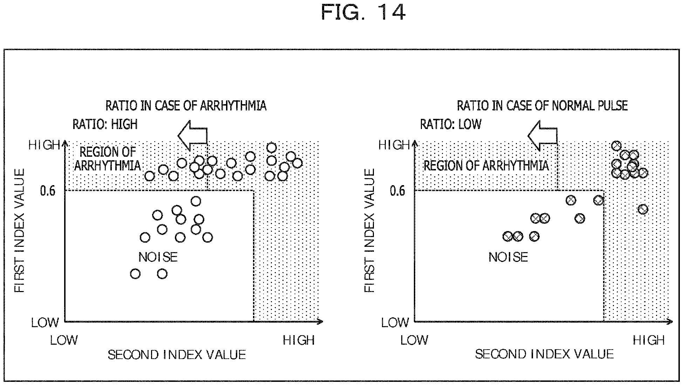

For example, the information processing apparatus 100 determines whether or not the acquired pulse wave waveforms at any part indicates an arrhythmia based on the calculated second index value in a case where it is determined that the noise is not included in the pulse wave waveform. Specifically, the information processing apparatus 100 determines that the pulse wave waveform of the face indicates an arrhythmia when the second index value is equal to or less than a second threshold value. The fact that the pulse wave waveform indicates an arrhythmia means that the pulse wave waveform includes a waveform in a case of arrhythmia. The details of determining whether or not to indicate an arrhythmia according to the first determination pattern will be described later with reference to FIGs. 13 and 14, for example.

<One Example of Determining whether or not Arrhythmia is indicated by Second Determination Pattern>

For example, the information processing apparatus 100 determines whether or not the acquired pulse wave waveforms at any part indicates an arrhythmia based on the calculated first index value, the calculated second index value, and information indicating the correspondence relationship of the first index value and the second index value in a case of an arrhythmia.

The information indicating the correspondence relationship is, for example, information for specifying a third threshold value for a value indicating the intensity of the correlation between the first index value and the second index value in a case of an arrhythmia. Specifically, the information processing apparatus 100 calculates a third index value indicating the intensity of the correlation between the calculated first index value and the calculated second index value, and determines that the pulse wave waveforms at any part indicates an arrhythmia when the third index value is equal to or less than the third threshold value.

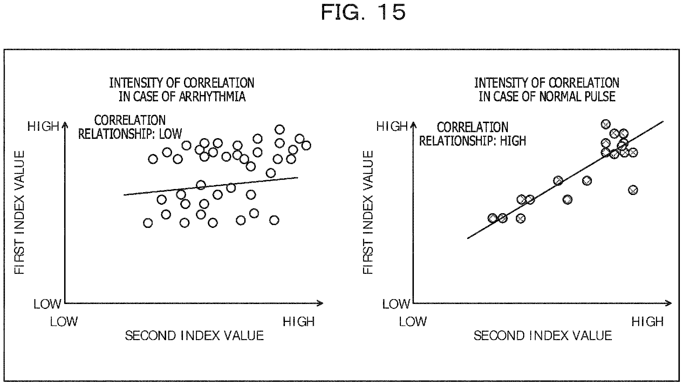

The information indicating the correspondence relationship may be information specifying a region where the combination of the first index value and the second index value in a case of an arrhythmia exists on a two-dimensional plane having an axis of the first index value and an axis of the second index value. When the calculated first index value is equal to or greater than the first index value that corresponds to the calculated second index value, the information processing apparatus 100 determines that the pulse wave waveforms at any part indicates an arrhythmia. The details of determining whether or not to indicate an arrhythmia according to the second determination pattern will be described later with reference to FIGs. 15 to 17, for example.

The information processing apparatus 100 outputs a determination result as to whether or not the pulse wave waveforms at any part indicates an arrhythmia. The information processing apparatus 100 correlates, for example, a pulse wave waveforms at any part with a determination result of whether or not the pulse wave waveforms at any part indicates an arrhythmia, displays the correlation result on a display of the information processing apparatus 100, and notifies the user of the information processing apparatus 100. According to this, the information processing apparatus 100 can calculate the first index value indicating the likelihood of the pulse wave of the pulse wave waveforms at any part of the subject based on the moving image 101 obtained by photographing the subject. In addition, the information processing apparatus 100 can calculate the second index value indicating the matching degree between the partial waveforms in the pulse wave waveforms at any part of the subject based on the moving image 101 obtained by photographing the subject.

Accordingly, the information processing apparatus 100 can determine whether or not the pulse wave waveforms at any part of the subject indicate an arrhythmia, based on the first index value and the second index value. In addition, the information processing apparatus 100 can improve the analysis accuracy of the pulse wave waveforms at any part of the subject. The information processing apparatus 100, for example, can distinguish whether the disturbance is caused by the noise caused by the body movement of the subject or the disturbance due to the arrhythmia even when the pulse wave waveforms at any part of the subject is disturbed. Further, for example, even when the noise is included in the pulse wave waveforms at any part of the subject, the information processing apparatus 100 can distinguish whether the pulse wave waveforms at any part of the subject is disturbed by the arrhythmia, or a waveform of a normal pulse. As a result, the information processing apparatus 100 can analyze the pulse wave waveform disturbed by the arrhythmia and make a determination on the health of the subject.

In this manner, the information processing apparatus 100 can improve the analysis accuracy of analyzing the pulse wave waveform obtained with respect to the subject even when the subject is not kept static. Therefore, the information processing apparatus 100 can efficiently measure the pulse wave waveform of the subject on a day-to-day basis continuously and without consciousness of the subject by the simplest possible method. As a result, the subject may not necessarily operate any device, and may not have to strive to keep static, and the burden when measuring the pulse wave waveform is reduced.

Here, a case where the information processing apparatus 100 acquires the pulse wave waveforms of each of the parts of the subject from another computer has been described, but the present invention is not limited thereto. For example, the information processing apparatus 100 may generate the pulse wave waveforms of each of the parts of the subject by photographing the subject and analyzing the moving image 101 obtained by photographing the subject. Specifically, the information processing apparatus 100 may operate as a server, may acquire the pulse wave waveforms of each of the parts of the subject from a client apparatus, and may determine whether or not to indicate the arrhythmia based on the acquired pulse wave waveforms of each of the parts. At this time, the information processing apparatus 100 may transmit a determination result as to whether or not the arrhythmia is indicated to the client apparatus.

Further, for example, by acquiring the moving image 101 obtained by photographing the subject from another computer and analyzing the moving image 101, the information processing apparatus 100 may generate the pulse wave waveforms of each of the parts of the subject. Specifically, the information processing apparatus 100 may operate as a server, may acquire the moving image 101 from the client apparatus, may generate the acquired pulse wave waveforms of each of the parts of the subject from the moving image 101, and may determine whether or not to indicate the arrhythmia based on the acquired pulse wave waveforms of each of the parts. At this time, the information processing apparatus 100 may transmit a determination result as to whether or not the arrhythmia is indicated to the client apparatus.

(Example of Hardware Configuration of Information Processing Apparatus 100)

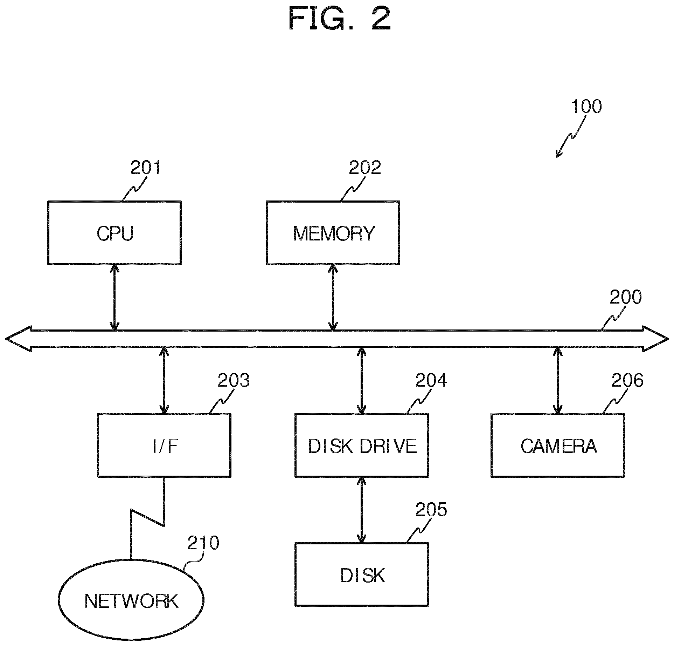

Next, an example of the hardware configuration of the information processing apparatus 100 illustrated in FIG. 1 will be described with reference to FIG. 2. The information processing apparatus 100 is, for example, a server, a personal computer (PC), a note PC, a tablet PC, or a smartphone.

FIG. 2 is a view illustrating an example of the hardware configuration of the information processing apparatus 100. In FIG. 2, the information processing apparatus 100 includes a central processing unit (CPU) 201, a memory 202, an interface (I/F) 203, a disk drive 204, a disk 205, and a camera 206. In addition, each of the components is connected to each other by a bus 200.

Here, the CPU 201 controls the entire information processing apparatus 100. The memory 202 includes, for example, a read only memory (ROM), a random access memory (RAM), and a flash ROM. Specifically, for example, the flash ROM or the ROM stores various programs, and the RAM is used as a work area of the CPU 201. The program stored in the memory 202 is loaded into the CPU 201, thereby causing the CPU 201 to execute the coded processing.

The I/F 203 is connected to a network 210 via a communication line, and is connected to another computer via the network 210. In addition, the I/F 203 controls the interface between the network 210 and the inside, and controls input and output of data from other computers. As the I/F 203, for example, a modem or a local area network (LAN) adapter can be adopted.

The disk drive 204 controls reading and writing of data from and to the disk 205 under the control of the CPU 201. The disk drive 204 is, for example, a magnetic disk drive. The disk 205 is a nonvolatile memory that stores data written under the control of the disk drive 204.

The camera 206 is a device having a photoelectric conversion element, such as a charge coupled device (CCD) or a complementary metal oxide semiconductor (CMOS). The camera 206 converts the optical signal from the subject within the photographing range into an electric signal by the photoelectric conversion element, generates image data from the converted electric signal, and stores the generated image data in the memory 202 or the disk 205. Specifically, the camera 206 is a front camera of a smartphone, a camera built in a PC, or the like.

In addition to the above-described components, the information processing apparatus 100 may have, for example, a solid state drive (SSD), a semiconductor memory, a keyboard, a mouse, and a display. Further, instead of the disk drive 204 and the disk 205, the information processing apparatus 100 may have an SSD, a semiconductor memory, and the like. In addition, the information processing apparatus 100 may not include the camera 206 and may be capable of communicating with another computer having a camera. Further, the information processing apparatus 100 may not include the camera 206 and may be capable of communicating with a fixed-point camera and the like existing on the network.

(Example of Functional Configuration of Information Processing Apparatus 100)

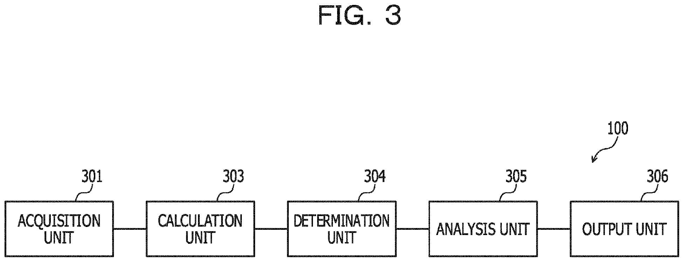

Next, an example of the functional configuration of the information processing apparatus 100 will be described with reference to FIG. 3. FIG. 3 is a block diagram illustrating an example of a functional configuration of the information processing apparatus 100. The information processing apparatus 100 includes an acquiring unit 301, a calculation unit 302, a determination unit 303, an analysis unit 304, and an output unit 305.

The acquiring unit 301 to the output unit 305 function as a control unit, and for example, by causing the CPU 201 to execute a program stored in a storage area, such as the memory 202 or the disk 205 illustrated in FIG. 2, or by using the I/F 203, the function thereof is realized. The processing result of each of the functional units is stored in a storage area, such as the memory 202 and the disk 205.

The acquiring unit 301 acquires the pulse wave waveforms of each of the parts obtained by analyzing the image of each of the parts of the plurality of parts of the subject included in each of the photographed images of the plurality of photographed images obtained by photographing the subject. The plurality of parts include, for example, two or more interlocking parts. Two or more interlocking parts are parts that move similarly according to the movement of the subject. Two or more interlocking parts are, for example, the left cheek and nose of the subject who moves similarly in accordance with the movement of the face of the subject.

In addition, the plurality of parts may include two or more parts which are not on the same plane. Two or more parts which are not on the same plane are, for example, two or more parts on a curved surface. Two or more parts not on the same plane are, for example, the left cheek and forehead of the subject. In addition, the plurality of parts may include parts symmetrically existing in the body of the subject. Symmetrically existing parts are, for example, the left cheek and the right cheek symmetrically existing on the face of the subject.

Two or more parts of the plurality of parts may be inclusive. The plurality of parts may include, for example, the face of the subject and the left cheek of the subject included in the face of the subject. Specifically, the plurality of parts may include the face of the subject, the left cheek of the face of the subject, and the right cheek of the face of the subject. The analysis means, for example, detecting a change in volume of blood that flows through the blood vessel of the part based on the change in skin color at the part by performing spectral analysis on a series of images of each of the parts of the subject.

The acquiring unit 301 receives, for example, the pulse wave waveforms of the face, the left cheek, and the right cheek of the subject from another computer. Accordingly, the acquiring unit 301 can acquire the pulse wave waveforms at any part of the subject who is the determination target of whether or not to indicate the arrhythmia. In addition, the acquiring unit 301 can acquire the pulse wave waveforms of each of the parts of the subject who is a calculation source of the first index value and the second index value which are used for determining whether or not to indicate the arrhythmia. Here, for example, a pulse wave waveform that serves as a determination target of whether or not to indicate an arrhythmia and a pulse wave waveform that serves as a calculation source of the first index value and the second index value may be the pulse wave waveform generated from at least the plurality of photographed images, may be a different pulse wave waveform, or may overlap each other.

The acquiring unit 301 may acquire the pulse wave waveforms of each of the parts in each of the periods obtained by analyzing the images of each of the parts included in each of the photographed images obtained by photographing the subjects in each of the periods within a predetermined period. Each of the periods may have a part that overlaps another period. Each of the periods may not be in contact with other periods and may be separated from other periods. Each of the periods may be continuous to other periods.

The acquiring unit 301 acquires, for example, the pulse wave waveforms of the face, the left cheek, and right cheek of the subject in each of the periods which are obtained by analyzing the images of the face, the left cheek, and the right cheek of the subject included in each of the photographed images obtained by photographing the subject in each of the periods within the predetermined period. Accordingly, the acquiring unit 301 can acquire the pulse wave waveforms at any part of the subject who is the determination target of whether or not to indicate the arrhythmia. In addition, the acquiring unit 301 can acquire the pulse wave waveforms of each of the parts of the subject who is a calculation source of the first index value and the second index value which are used for determining whether or not to indicate the arrhythmia.

The acquiring unit 301 may acquire each of the photographed images of the plurality of photographed images obtained by photographing the subject, may analyze the images of each of the parts of the subject included in each of the photographed images, and may generate the pulse wave waveforms of each of the parts. The plurality of photographed images may be, for example, frame images that satisfy a predetermined condition extracted from a series of frame images included in the moving image 101. More specifically, the plurality of photographed images may be a plurality of frame images photographed at intervals of 1/6 seconds out of a series of frame images photographed at intervals of 1/60 seconds.

The plurality of photographed images may be, for example, a series of processed frame images obtained by processing a series of frame images included in the moving image 101. Specifically, the plurality of photographed images may be a series of images represented by predetermined wavelength components obtained by extracting a predetermined wavelength component from a series of frame images included in the moving image 101. The plurality of photographed images may be a plurality of images successively photographed by a photographing apparatus, such as a digital camera capable of photographing a still image.

The image at any part may not have a fixed size in each of the photographed images. Each of the photographed images may be, for example, a photographed image in which an image at any part of the subject extracted from the moving image 101 becomes equal to or greater than a predetermined size. The moving image 101 is, for example, a series of frame images photographed by using the photographing apparatus, such as a video camera capable of photographing a video by another computer.

For example, the acquiring unit 301 acquires the moving image 101 obtained by photographing the subject and extracts the images of the face, the left cheek, and the right cheek of the subject included in each of the photographed images of the acquired moving image 101. In addition, the acquiring unit 301 generates the pulse wave waveforms of the face, the left cheek, and the right cheek of the subject by analyzing the extracted image. Accordingly, the acquiring unit 301 can acquire the pulse wave waveforms at any part of the subject who is the determination target of whether or not to indicate the arrhythmia. In addition, the acquiring unit 301 can acquire the pulse wave waveforms of each of the parts of the subject who is a calculation source of the first index value and the second index value which are used for determining whether or not to indicate the arrhythmia.

The acquiring unit 301 may acquire each of the photographed images obtained by photographing the subject in each of the periods within the predetermined period. In addition, the acquiring unit 301 may analyze the images of each of the parts of the subject included in each of the acquired photographed images in each of the periods, and may generate the pulse wave waveforms of each of the parts in each of the periods.

For example, the acquiring unit 301 acquires each of the photographed images obtained by photographing the subjects photographed in each of the periods within the predetermined period, and extracts the images of the face, the right cheek, and the left cheek of the subject included in each of the acquired photographed images in each of the periods. In addition, the acquiring unit 301 generates the pulse wave waveforms of the face, the left cheek, and the right cheek of the subject in each of the periods by analyzing the images of the face, the left cheek, and the right cheek of the subject extracted from each of the photographed images in each of the periods. Accordingly, the acquiring unit 301 can acquire the pulse wave waveforms at any part of the subject who is the determination target of whether or not to indicate the arrhythmia. In addition, the acquiring unit 301 can acquire the pulse wave waveforms of each of the parts of the subject who is a calculation source of the first index value and the second index value which are used for determining whether or not to indicate the arrhythmia.

The acquiring unit 301 may photograph the subject, may acquire each of the photographed images obtained by photographing the subject, may analyze the images of each of the parts of the subject included in each of the photographed images, and may generate the pulse wave waveforms of each of the parts.

For example, the acquiring unit 301 photographs the subject by using the camera 206, acquires the moving image 101 obtained by photographing the subject, and extracts the images of the face, the left cheek, and the right cheek of the subject included in each of the photographed images of each of the acquired moving image 101. In addition, the acquiring unit 301 generates the pulse wave waveforms of the face, the left cheek, and the right cheek of the subject by analyzing the extracted image. Accordingly, the acquiring unit 301 can acquire the pulse wave waveforms at any part of the subject who is the determination target of whether or not to indicate the arrhythmia. In addition, the acquiring unit 301 can acquire the pulse wave waveforms of each of the parts of the subject who is a calculation source of the first index value and the second index value which are used for determining whether or not to indicate the arrhythmia.

The acquiring unit 301 may photograph the subject in each of the periods within the predetermined period, and may acquire each of the photographed images obtained by photographing the subject. In addition, the acquiring unit 301 may analyze the images of each of the parts of the subject included in each of the acquired photographed images in each of the periods, and may generate the pulse wave waveforms of each of the parts.

For example, the acquiring unit 301 photographs the subject in each of the periods within the predetermined period, acquires each of the photographed images obtained by photographing the subjects in each of the periods, and extracts the images of the face, the right cheek, and the left cheek of the subject included in each of the acquired photographed images in each of the periods. In addition, the acquiring unit 301 generates the pulse wave waveforms of the face, the left cheek, and the right cheek of the subject in each of the periods by analyzing the images of the face, the left cheek, and the right cheek of the subject extracted from each of the photographed images in each of the periods.

Accordingly, the acquiring unit 301 can acquire the pulse wave waveforms at any part of the subject who is the determination target of whether or not to indicate the arrhythmia. In addition, the acquiring unit 301 can acquire the pulse wave waveforms of each of the parts of the subject who is a calculation source of the first index value and the second index value which are used for determining whether or not to indicate the arrhythmia.

The calculation unit 302 calculates the first index value indicating the likelihood of pulse waves of the pulse wave waveforms at any part of the subject. The first index value is, for example, a value indicating the matching degree between at least one of the pulse wave waveforms of the pulse wave waveforms of each of the parts of the subject. The first index value may be a statistical value, such as an average value, a median value, a maximum value, and a minimum value of values indicating the matching degree between the pulse wave waveforms of the pulse wave waveforms of each of the parts of the subject.

For example, the calculation unit 302 calculates a value indicating the matching degree between the pulse wave waveforms of the pulse wave waveforms of each of the parts acquired by the acquiring unit 301 as the first index value. Specifically, by using the correlation coefficient, the calculation unit 302 calculates a value indicating the matching degree between the pulse wave waveforms of the left cheek and right cheek of the pulse wave waveforms of the face, the left cheek, and the right cheek of the subject acquired by the acquiring unit 301, as the first index value. Accordingly, the calculation unit 302 can calculate the first index value used for determining whether or not to indicate an arrhythmia.

For example, for each of the periods, the calculation unit 302 may calculate a value indicating the matching degree between the pulse wave waveforms of the pulse wave waveforms of each of the parts in each of the periods acquired by the acquiring unit 301, as the first index value. Specifically, by using the correlation coefficient, for each of the periods, the calculation unit 302 calculates a value indicating the matching degree between the pulse wave waveforms of the left cheek and the right cheek of the pulse wave waveforms of the face, the left cheek, and the right cheek of the subject in each of the periods acquired by the acquiring unit 301, as the first index value. Accordingly, the calculation unit 302 can calculate the first index value used for determining whether or not to indicate an arrhythmia.

The calculation unit 302 calculates the second index value indicating the matching degree between the partial waveforms of the pulse wave waveforms at any part of the subject. For example, the second index value is a value indicating the matching degree between partial waveforms having a length that corresponds to a predetermined cycle in the pulse wave waveforms of each of the parts of the subject. The second index value may be a statistical value, such as an average value, a median value, a maximum value, and a minimum value of values indicating the matching degree between the partial waveforms having the length that corresponds to the predetermined cycle in the pulse wave waveforms of each of the parts of the subject.

The second index value may be a value indicating the magnitude of the variation in the pulse wave interval of the pulse wave waveforms at any part of the subject. The second index value may be a statistical value, such as an average value, a median value, a maximum value, and a minimum value of values indicating the magnitude of the variation in the pulse wave interval of the pulse wave waveforms at each of the parts of the subject. The second index value may be a value indicating the magnitude of the variation in the amplitude of the pulse wave waveforms at any part of the subject. The second index value may be a statistical value, such as an average value, a median value, a maximum value, and a minimum value of values indicating the magnitude of the variation in the amplitude of the pulse wave waveforms at each of the parts of the subject.

For example, the calculation unit 302 calculates the second index value indicating the matching degree between the partial waveforms of the pulse wave waveforms of at least one of the parts, in the pulse wave waveforms at each of the parts acquired by the acquiring unit 301, as the second index value. Specifically, by using the correlation coefficient, the calculation unit 302 calculates a value indicating the matching degree between the partial waveforms of the pulse wave waveform of the face of the subject acquired by the acquiring unit 301, as the second index value. Accordingly, the calculation unit 302 can calculate the second index value used for determining whether or not to indicate an arrhythmia.

For example, for each of the periods, the calculation unit 302 may calculate a value indicating the matching degree between the partial waveforms of the pulse wave waveforms at any part in each of the periods acquired by the acquiring unit 301, as the second index value. Specifically, by using the correlation coefficient, for each of the periods, the calculation unit 302 calculates a value indicating the matching degree between the partial waveforms of the pulse wave waveforms of the face of the subject in each of the periods acquired by the acquiring unit 301, as the second index value. Accordingly, the calculation unit 302 can calculate the second index value used for determining whether or not to indicate an arrhythmia.

The calculation unit 302 calculates the third index value based on the first index value and the second index value calculated by the calculation unit 302 for each of the periods. The third index value is a value indicating the intensity of the correlation between the first index value and the second index value. For example, the third index value is a value of a correlation coefficient obtained from the first index value and the second index value calculated by the calculation unit 302 for each of the periods. For example, the calculation unit 302 calculates a value that corresponds to the first index value and the second index value calculated by the calculation unit 302 by substituting the first index value and the second index value calculated by the calculation unit 302 for each of the periods in the calculation formula of the correlation coefficient, as the third index value. Accordingly, the calculation unit 302 can calculate the third index value used for determining whether or not to indicate an arrhythmia.

The calculation unit 302 calculates a fourth index value based on the second index value calculated for each of the periods. The fourth index value may be a value indicating the magnitude of the variation in the size of disturbance of the pulse wave waveforms at any part of the subject. The fourth index value is a value indicating the magnitude of the variation of the second index value. The fourth index value is, for example, a difference between the maximum value and the minimum value of the second index value. The fourth index value may be a standard deviation of the second index value. The fourth index value may be a median value of the second index value. Accordingly, the calculation unit 302 can calculate the fourth index value used for determining whether or not to indicate an arrhythmia.

In a case of calculating the fourth index value, the calculation unit 302 may not calculate the third index value when the calculated fourth index value is equal to or less than the fourth threshold value. In addition, when the calculated fourth index value is greater than the fourth threshold value, the calculation unit 302 calculates the third index value. Accordingly, the calculation unit 302 can reduce the processing amount.

The determination unit 303 determines whether or not the pulse wave waveforms at any part of the subject indicate an arrhythmia according to the first determination pattern or the second determination pattern. Further, the determination unit 303 may selectively use the first determination pattern and the second determination pattern based on the fourth index value. In the following description, a case where the first determination pattern and the second determination pattern are used differently may be referred to as "third determination pattern" in some cases.

< First Determination Pattern>

Based on the first index value calculated by the calculation unit 302 for each of the periods, the determination unit 303 determines whether or not the noise is included in the pulse wave waveforms at any part in each of the periods acquired by the acquiring unit 301. For example, for each of the periods, in a case where the first index value calculated by the calculation unit 302 is equal to or less than the first threshold value, the determination unit 303 determines that the noise is included in the pulse wave waveform of the face of the subject in each of the periods acquired by the acquiring unit 301.

Based on the first index value and the second index value calculated by the calculation unit 302 for each of the periods, the determination unit 303 may determine whether or not the noise is included in the pulse wave waveforms at any part in each of the periods acquired by the acquiring unit 301. For example, for each of the periods, in a case where the first index value calculated by the calculation unit 302 is equal to or less than the first threshold value, the determination unit 303 determines that the noise is included in the pulse wave waveform of the face of the subject in each of the periods acquired by the acquiring unit 301. In addition, for each of the periods, in a case where the second index value calculated by the calculation unit 302 is equal to or less than a fifth threshold value, the determination unit 303 determines that the noise is included in the pulse wave waveform of the face of the subject in each of the periods acquired by the acquiring unit 301. The fifth threshold value is, for example, a value greater than the second threshold value.

Based on the second index value calculated by the calculation unit 302 for each of the periods during which it is determined that the noise is not included within the predetermined period, the determination unit 303 determines whether or not the pulse wave waveforms at any part of the subject indicate an arrhythmia for each of the periods during which it is determined that the noise is not included.

The determination unit 303 determines whether or not the second index value calculated by the calculation unit 302 is smaller than the second threshold value for each of the periods during which it is determined that the noise is not included within the predetermined period. When there is a period during which it is determined that the second index value calculated by the calculation unit 302 is smaller than the second threshold value, the determination unit 303 determines that the pulse wave waveform of the face of the subject in the period during which it is determined that the second index value is smaller than the second threshold value indicates an arrhythmia.

In determining whether or not the noise is included, the determination unit 303 employs a value indicating the matching degree between the partial waveforms as the second index value, and in the determination as to whether or not to indicate an arrhythmia, the determination unit 303 may employ a value indicating the magnitude of the variation in the pulse wave interval as the second index value. In addition, in determining whether or not the noise is included, the determination unit 303 employs a value indicating the magnitude of the variation in the pulse wave interval as the second index value, and in the determination as to whether or not to indicate an arrhythmia, the determination unit 303 may employ a value indicating the matching degree between the partial waveforms as the second index value. Accordingly, the determination unit 303 can detect the pulse wave waveforms at any part of the subject indicating the arrhythmia, in the pulse wave waveforms at any part of the subject in each of the periods.

Based on the second index value calculated by the calculation unit 302 for each of the periods during which it is determined that the noise is not included within the predetermined period, the determination unit 303 determines whether or not the pulse wave waveforms at any part of the subject indicate an arrhythmia in the predetermined period.

For example, the determination unit 303 determines whether or not the second index value calculated by the calculation unit 302 is smaller than the second threshold value for each of the periods during which it is determined that the noise is not included within the predetermined period. Based on a ratio of the period during which it is determined that the second index value is smaller than the second threshold value in the period during which it is determined that the noise is not included within the predetermined period, the determination unit 303 determines whether or not the pulse wave waveforms at any part within the predetermined period indicates an arrhythmia. Accordingly, the determination unit 303 can determine whether or not the pulse wave waveforms at any part of the subject in a predetermined period indicate an arrhythmia.

Based on the determination result as to whether or not the pulse wave waveforms at any part of the subject in each of the periods during which it is determined that the noise is not included within the predetermined period indicate the arrhythmia, the determination unit 303 may generate information indicating the correspondence relationship between the first index value and the second index value in a case of an arrhythmia. Accordingly, the determination unit 303 can generate information indicating the correspondence relationship used for the second determination pattern.

<Second Determination Pattern>

Based on the combination of the first index value and the second index value calculated by the calculation unit 302 and the information indicating the correspondence relationship between the first index value and the second index value, the determination unit 303 determines whether or not the acquired pulse wave waveforms at any part indicates an arrhythmia. The information indicating the correspondence relationship is, for example, information on a value indicating the intensity of the correlation between the first index value and the second index value in a case of an arrhythmia. The information indicating the correspondence relationship may be information of a region where the combination of the first index value and the second index value in a case of an arrhythmia exists on a two-dimensional plane having an axis of the first index value and an axis of the second index value, or may be information specifying the region. The information indicating the correspondence relationship may be information on the combination of the first index value and the second index value in a case where the pulse wave waveform is not an arrhythmia. Otherwise, the information indicating the correspondence relationship may be information on the combination of the first index value and the second index value both in a case of an arrhythmia and in a case where the pulse wave waveform is not an arrhythmia.

The determination unit 303, for example, refers to the information indicating the correspondence relationship and specifies the first index value that corresponds to the second index value calculated by the calculation unit 302 for each of the periods. In a case where it is determined that the first index value calculated by the calculation unit 302 for each of the period is equal to or greater than the specified first index value, the determination unit 303 determines that the acquired pulse wave waveforms at any part indicates an arrhythmia. Accordingly, the determination unit 303 can determine whether or not the pulse wave waveforms at any part of the subject in each of the periods indicate an arrhythmia.

For example, by using the information indicating the correspondence relationship as the third index value, in a case where it is determined that the third index value calculated by the calculation unit 302 is equal to or less than the third threshold value, the determination unit 303 determines that the pulse wave waveforms at any part within the predetermined period indicate an arrhythmia. Accordingly, the determination unit 303 can determine whether or not the pulse wave waveforms at any part of the subject in a predetermined period indicate an arrhythmia.

<Third Determination Pattern>

In a case where the fourth index value calculated by the calculation unit 302 is equal to or less than the fourth threshold value, by the first determination pattern, the determination unit 303 determines whether or not the pulse wave waveforms at any part in each of the periods indicate an arrhythmia. In addition, in a case where the fourth index value calculated by the calculation unit 302 is equal to or less than the fourth threshold value, by the first determination pattern, the determination unit 303 determines whether or not the pulse wave waveforms at any part within the predetermined period indicate an arrhythmia. Accordingly, the determination unit 303 can determine whether or not the pulse wave waveforms at any part of the subject in each of the periods indicate an arrhythmia. Further, the determination unit 303 can determine whether or not the pulse wave waveforms at any part of the subject in the predetermined period indicate an arrhythmia.

In a case where the fourth index value calculated by the calculation unit 302 is equal to or greater than the fourth threshold value, by the second determination pattern, the determination unit 303 determines whether or not the pulse wave waveforms at any part in each of the periods indicate an arrhythmia. In addition, in a case where the fourth index value calculated by the calculation unit 302 is equal to or greater than the fourth threshold value, by the second determination pattern, the determination unit 303 determines whether or not the pulse wave waveforms at any part within the predetermined period indicate an arrhythmia. Accordingly, the determination unit 303 can determine whether or not the pulse wave waveforms at any part of the subject in each of the periods indicate an arrhythmia. Further, the determination unit 303 can determine whether or not the pulse wave waveforms at any part of the subject in the predetermined period indicate an arrhythmia.

The analysis unit 304 analyzes the pulse wave waveforms at any part of the subject based on the determination result as to whether or not the pulse wave waveforms at any part of the subject indicate an arrhythmia. The analysis unit 304 calculates, for example, an index value related to analysis of the pulse wave waveform of the subject who is determined to exhibit the arrhythmia. Specifically, the analysis unit 304 calculates an index value of stress applied to the subject by using the pulse wave waveforms at any part of the subject who is determined to exhibit the arrhythmia, or determines whether or not there is a sign of disease, such as cerebral infarction. Accordingly, the analysis unit 304 can analyze the pulse wave waveform of the subject and make a determination regarding the health of the subject.

The output unit 305 outputs at least one of a determination result as to whether or not the pulse wave waveforms at any part indicates an arrhythmia or an analysis result by the analysis unit 304. The output format includes, for example, display on a display, printout to a printer, transmission to an external device by the I/F 203, storage in a storage region, such as the memory 202 or the disk 205, and the like.

The output unit 305 outputs, for example, the pulse wave waveform of the face of the subject acquired by the acquiring unit 301 and the determination result of whether or not the pulse wave waveform of the face of the subject indicates an arrhythmia in association with each other. Accordingly, the output unit 305 can notify the user of the determination result as to whether or not the pulse wave waveform of the face of the subject indicates an arrhythmia. The user can refer to the determination result as to whether or not the pulse wave waveform of the face of the subject indicates an arrhythmia, and can make a determination regarding the health of the subject.

(Example for Determining whether or not Pulse Wave Waveform of Subject Indicates Arrhythmia)

Next, with reference to FIGs. 4 to 17, an example will be described in which the information processing apparatus 100 determines whether or not the pulse wave waveform of the subject indicates an arrhythmia.

First, the information processing apparatus 100 acquires the moving image 101 obtained by photographing the subject within the predetermined period. The moving image 101 is, for example, a series of photographed images expressed by the luminance of RGB. Further, the moving image 101 may be expressed by luminance of a color belonging to a wavelength band different from that of RGB. In addition, the moving image 101 may be expressed by near-infrared or red brightness.

Further, the moving image 101 may be, for example, a series of data correlated with luminance. As for the moving image 101, for example, similar to a series of data acquired by a laser speckle blood flow meter, when the signal intensity varies depending on how the light hits, the signal intensity may depend on the amount and the speed of red blood cells. The information processing apparatus 100 may acquire the plurality of moving images 101 obtained by photographing the subject within the predetermined period.

Next, the information processing apparatus 100 extracts a predetermined image of the subject from the acquired moving image 101. The predetermined image is, for example, an image in which a part of the body of the subject including a plurality of parts of the subject is taken. A part of the body of the subject is, for example, a face, an arm, a neck, an ankle, a hand, a finger, and the like. For example, when a part of the body of the subject is a face, the plurality of parts are a face, a left side of the face, a right side of the face, a forehead, a left cheek, a right cheek, and the like.

For example, similar to any of the first to third examples of extracting the images of each of the parts of the subject, which will be described later with reference to FIGs. 4 to 6, the information processing apparatus 100 extracts the images of each of the parts of the subject who is the generation source of the pulse wave waveforms of each of the parts of the subject from the acquired moving image 101. Here, the description of FIG. 4 will be made.

<First Example of Extracting Images of Each of Parts of Subject>

FIG. 4 is an explanatory view illustrating a first example of extracting the images of each of the parts of the subject. Here, in the information processing apparatus 100, it becomes easy to measure the pulse wave waveform with high accuracy when the region where the body of the subject appears in the image of the generation source of the pulse wave waveform is large. Meanwhile, in the information processing apparatus 100, it becomes difficult to measure the pulse wave waveform with high accuracy when the region other than the body of the subject is included in the image of the generation source of the pulse wave waveform. For example, since a region, such as a background where no subject is photographed, exhibits luminance other than the part of the subject, it is possible to generate the noise to the pulse wave waveform of the part of the subject.

Therefore, it is preferable that the information processing apparatus 100 extracts an image of each of the parts of the subject such that the region where each of the parts of the subject appears becomes large and the region where the part other than each of the parts of the subject appears becomes small. Further, the information processing apparatus 100 may not necessarily extract the images of each of the parts of the subject from all of the photographed images in the moving image 101. For example, when the region where each of the parts of the subject appears in any one of the photographed images of the moving image 101 is equal to or less than a certain value, the information processing apparatus 100 may not extract the image of each of the parts of the subject from the photographed image.

In FIG. 4, for each photographed image 400 of the moving image 101, the information processing apparatus 100 detects a region where the face of the subject is taken by using a face detection engine, and extracts an image 410 of the face of the subject as one image of the part of the subject. The face detection engine is software for detecting a human face and a region of the human face where parts, such as a forehead, a nose, a mouth, eyes, or cheeks, from a predetermined image. The information processing apparatus 100 further divides the image 410 of the face of the subject extracted from each of the photographed images 400 of the moving image 101 into left and right images, and extracts an image 411 on the left side of the face as one of the images of the part of the subject and an image 412 on the right side of the face.

Here, there is a tendency that the S (signal)/N (noise) ratio becomes high in the pulse wave waveform generated from the image in which the region where the part of the subject is taken is relatively large. The S/N ratio indicates that the noise is relatively small when the value is relatively high. Therefore, the information processing apparatus 100 sets to use the image of the face of the subject in which the region where the part of the subject is taken is relatively large, as the image of the generation source of the pulse wave waveform to be determined whether or not to indicate the arrhythmia. In addition, the information processing apparatus 100 sets to use the remaining left image 411 on the left side of the face and the image 412 on the right side of the face, as the image of the generation source of the pulse wave waveform used for calculating the first index value.

At this time, the information processing apparatus 100 may not necessarily extract the image 410 of the face of the subject, the image 411 on the left side of the face, and the image 412 on the right side of the face from all of the photographed images 400 among the photographed images 400 included in the moving image 101. For example, the information processing apparatus 100 may not extract the image 411 on the left side of the face and the image 412 on the right side of the face from the photographed image 400 in which the image 411 on the left side of the face or the image 412 on the right side of the face is equal to or less than the predetermined size.

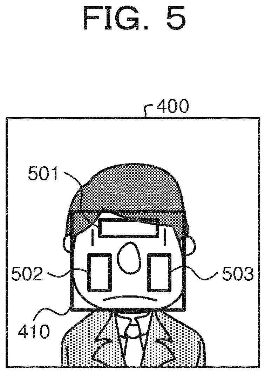

Accordingly, the information processing apparatus 100 can make it easy to measure the pulse wave waveforms of each of the parts of the subject with high accuracy without extracting the image of each of the parts of the subject from the photographed image 400 which has a possibility of deterioration of the accuracy of measuring the pulse wave waveform. In addition, the information processing apparatus 100 can reduce the amount of processing demanded for extracting the image of each of the parts of the subject and suppress the increase in processing time. Here, the description of FIG. 5 will be made.

<Second Example of Extracting Images of Each of Parts of Subject>

FIG. 5 is an explanatory view illustrating a second example of extracting the images of each of the parts of the subject. Here, by using the images of each of the parts of three or more parts of the subject as the image of the generation source of the pulse wave waveform used for calculating the first index value, the information processing apparatus 100 may calculate the first index value in consideration of various body movements of the subject. Various body movements are, for example, up and down movements of face of the subject and left and right movements of the face of the subject.

For example, the information processing apparatus 100 includes an image of a part existing in the lateral direction of the face of the subject as the image of the generation source of the pulse wave waveform used for calculating the first index value, and includes the image of the part existing in the vertical direction of the face of the subject. The image of the part existing in the lateral direction of the face of the subject and the image of the part existing in the vertical direction of the face of the subject may include overlapping images. Accordingly, the information processing apparatus 100 can calculate the first index value in more consideration of the noise caused by the left and right movement of the face of the subject and the noise caused by the up and down movement of the face of the subject.

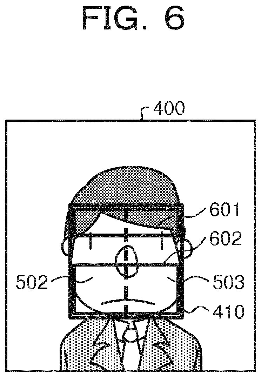

In FIG. 5, for each of the photographed images 400 of the moving image 101, the information processing apparatus 100 detects the region where the face of the subject is taken by using the face detection engine, and extracts the image 410 of the face of the subject. By using the face detection engine, the information processing apparatus 100 further detects a region where the forehead of the subject is taken, a region where the left cheek is taken, and a region where the right cheek is taken, and extracts an image 501 of the forehead of the subject, an image 502 of the left cheek, and an image 503 of the right cheek. Accordingly, the information processing apparatus 100 can calculate the first index value in more consideration of the up and down movement of the face or the like other than the left and right movement of the face. Here, the description of FIG. 6 will be made.

<Third Example of Extracting Images of Each of Parts of Subject>

FIG. 6 is an explanatory view illustrating a third example of extracting the images of each of the parts of the subject. Here, in the body of the subject, a part at which it is difficult to keep a state where the subject is static and it is easy to generate the noise in the pulse wave waveform, exists. For example, it is difficult to keep a state where the eyes of the subject are static for a long period of time due to blinking, and there is a tendency that the noise is generated in the pulse wave waveform.

Therefore, as the image of the generation source of the pulse wave waveforms of each of the parts of the subject, the information processing apparatus 100 may extract an image excluding the region where it is difficult to keep a state where the subject is static and the part at which the noise is likely to be generated in the pulse wave waveform is taken. Accordingly, the information processing apparatus 100 can easily calculate the first index value with high accuracy.

In FIG. 6, for each of the photographed images 400 of the moving image 101, the information processing apparatus 100 detects the region where the face of the subject is taken by using the face detection engine, and extracts the image 410 of the face of the subject. By using the face detection engine, the information processing apparatus 100 further detects the region where the eyes of the subject are taken and excludes the region where the eyes of the subject are taken from the region where the face of the subject is taken.

Further, the information processing apparatus 100 further extracts the image 601 of the upper part of the face above the region where the eyes of the subject are taken, in the image 410 of the face of the subject. Further, the information processing apparatus 100 further extracts an image 602 of the lower part of the face below the region where the eyes of the subject are taken, in the image 410 of the face of the subject. In addition, the information processing apparatus 100 further extracts the image 502 of the left cheek and the image 503 of the right cheek in the image 602 of the lower part of the face of subject.

Accordingly, the information processing apparatus 100 can extract an image excluding a region where a part at which the noise of the eyes or the like is likely to be generated is taken, and reduce the noise. Hereinafter, similar to the first example illustrated in FIG. 4, the description of the example will continue using a case where the information processing apparatus 100 extracts the image 410 of the face of the subject, the image 411 on the left side of the face of the subject, and the image 412 on the right side of the face of the subject as an example.

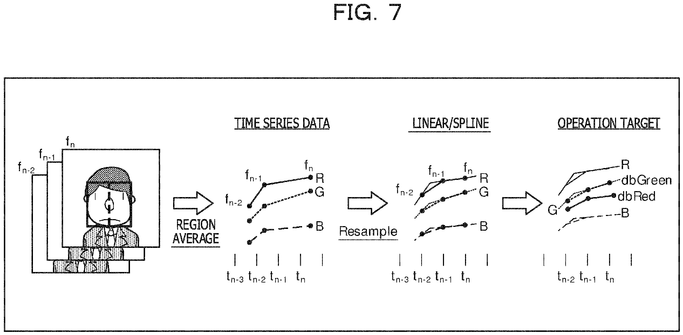

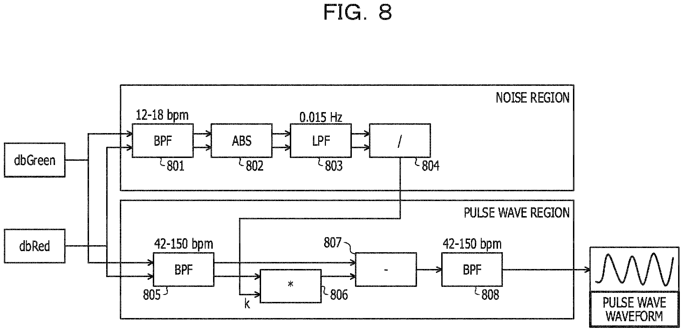

Based on the extracted image 410 of the face of the subject, the image 411 on the left side of the face, and the image 412 on the right side of the face, the information processing apparatus 100 generates the pulse wave waveform of the face of the subject, the pulse wave waveform on the left side of the face, and the pulse wave waveform on the right side of the face. The information processing apparatus 100 generates the pulse wave waveform of the face of the subject, the pulse wave waveform on the left side of the face, and the pulse wave waveform on the right side of the face, for example, similar to one example which will be described in FIGs. 7 and 8 later. Here, the description of FIG. 7 will be made.

<One Example of Generating Pulse Wave Waveforms of Each of Parts of Subject>

FIGs. 7 and 8 are explanatory views illustrating an example of generating the pulse wave waveforms of each of the parts of the subject. As a technology for generating the pulse wave waveform, for example, it is possible to refer to the technology described in International Publication Pamphlet No. WO 2014/038077.

Here, depending on the contraction or expansion of the heart, the flow rate of the blood that flows through the blood vessel of the human body varies. In addition, the amount of light absorbed by the blood vessel of the subject tends to depend on the flow rate of blood that flows through the blood vessel. Therefore, in accordance with the contraction or expansion of the heart, the luminance of the image of the part of the subject in the photographed image also changes, and the luminance that corresponds to the pulse at the part of the subject is obtained. In other words, the time series data of the representative value of the predetermined wavelength component in the image of the part of the subject in the photographed image becomes the time series data including the pulse component of the part of the subject.

Meanwhile, there is a case where the luminance of the image of the part of the subject in the photographed image changes in accordance with the change in light amount that corresponds to the part of the subject due to the body movement or the like of the subject. Therefore, there is a case where the time series data of the representative value of the predetermined wavelength component in the image of the part of the subject in the photographed image also includes a noise component different from the pulse component for the part of the subject.

Here, the information processing apparatus 100 acquires the time series data of the representative values of the plurality of wavelength components, performs a predetermined operation using a filter or the like which will be described later, reduces the noise component from the time series data of the representative value of the predetermined wavelength component of the photographed image, and generate the pulse wave waveform of the part of the subject.

In FIG. 7, based on the images at any part of the subject extracted from each of the photographed images fn-2, fn-1, fn and the like, the information processing apparatus 100 acquires the time series data of the representative value of the predetermined wavelength component included in the image. For example, the information processing apparatus 100 acquires the time series data of the representative values of an R component, a G component, and a B component included in the image at any part of the subject. The representative values of the R component, the G component, and the B component are, for example, average values of the R component, the G component, and the B component, respectively.

In a case where the time series data of the acquired representative value is not the time series data of an equal time interval, the information processing apparatus 100 converts the time series data of the representative value into the time series data of the equal time interval by a resampling technology that uses linear approximation or spline approximation. The time series data at equal time intervals are time series data of the representative values of the predetermined wavelength components at each of the times tn-2, tn-1, tn, and the like, for example.

Since the hemoglobin in the blood has characteristics of absorbing green light, the information processing apparatus 100 sets the time series data of the representative value of the G component having a relatively high degree of representing the pulse component as dbGreen. In addition, the information processing apparatus 100 generates the time series data of the average value of the representative value of the R component and the representative value of the B component other than the representative value of the G component, and sets the time series data as dbRed. Here, the description of FIG. 8 will be made.

In FIG. 8, the information processing apparatus 100 extracts signal components in a specific frequency band that does not include a heartbeat component from the dbGreen and the dbRed by a band pass filter (BPF). The specific frequency band is, for example, a frequency of 12 bpm or greater and less than 18 bpm. In addition, the information processing apparatus 100 calculates the ratio of the noise component used as a correction coefficient k when generating the pulse wave waveform, based on the signal component of the extracted specific frequency band.

In addition, the information processing apparatus 100 extracts the signal components of a pulse wave band that includes a heartbeat component from the dbGreen and the dbRed by the BPF. The pulse wave frequency band is, for example, a frequency of 42 bpm or greater and less than 150 bpm. In addition, the information processing apparatus 100 generates the pulse wave waveforms at any part of the subject based on the extracted signal component of the pulse wave frequency band and the correction coefficient k.

Specifically, the information processing apparatus 100 extracts the signal component of the specific frequency band of the dbRed by a BPF 801. In addition, the information processing apparatus 100 extracts the signal component of the specific frequency band of the dbGreen by the BPF 801. Next, the information processing apparatus 100 extracts the time series data of the absolute value of the signal component of the specific frequency band of the dbRed by an operation unit 802. In addition, the information processing apparatus 100 extracts the time series data of the absolute value of the signal component of the specific frequency band of the dbGreen by an operation unit 802.

In addition, the information processing apparatus 100 performs a smoothing process on the time series data of the absolute value of the specific frequency band of dbRed by a low pass filter (LPF) 803, and removes steep frequency components. In addition, the information processing apparatus 100 performs a smoothing process with respect to the time series data of the absolute value of the specific frequency band of the dbGreen by the LPF 803, and removes steep frequency components. Furthermore, the information processing apparatus 100 calculates the correction coefficient k by the operation unit 804 by dividing the absolute value of the specific frequency band of the smoothed dbRed by the absolute value of the specific frequency band of the smoothed dbGreen.

The information processing apparatus 100 extracts the signal component of the pulse wave frequency band of the dbRed by a BPF 805. In addition, the information processing apparatus 100 extracts the signal component of the pulse wave frequency band of the dbGreen by the BPF 805. Next, in the information processing apparatus 100, an operation unit 806 multiplies the signal component in the pulse wave frequency band of the extracted dbRed by the calculated correction coefficient k. In addition, in the information processing apparatus 100, an operation unit 807 subtracts the signal component of the pulse wave frequency band of the dbRed multiplied by the correction coefficient k from the signal component of the pulse wave frequency band of the extracted dbGreen.

Next, the information processing apparatus 100 extracts the signal component of the pulse wave frequency band of the time series data of the signal after the subtraction by a BPF 808. In addition, the information processing apparatus 100 sets the extracted waveform as the pulse wave waveforms at any part of the subject. In addition, the information processing apparatus 100 performs the process similar to that in FIGs. 7 and 8 for each of the images of the remaining parts, and sets the pulse wave waveforms of the remaining parts.

Here, a case where the information processing apparatus 100 acquires the time series data of the representative values of the plurality of wavelength components has been described, but the present invention is not limited thereto. For example, the information processing apparatus 100 may extract the signal component in the pulse wave frequency band from the time series data of the representative value of the predetermined wavelength component by the BPF, and may set the extracted waveform as the pulse wave waveforms at any part of the subject.

Here, a case where the information processing apparatus 100 generates the pulse wave waveform based on the luminance that corresponds to the flow rate of the blood has been described, but the present invention is not limited thereto. For example, the information processing apparatus 100 may generate the pulse wave waveform by detecting a change of the skin of the subject due to contraction or expansion of the heart. In addition, for example, the information processing apparatus 100 may generate the pulse wave waveform by detecting the blood vessel or the movement of the blood of the subject due to contraction or expansion of the heart.

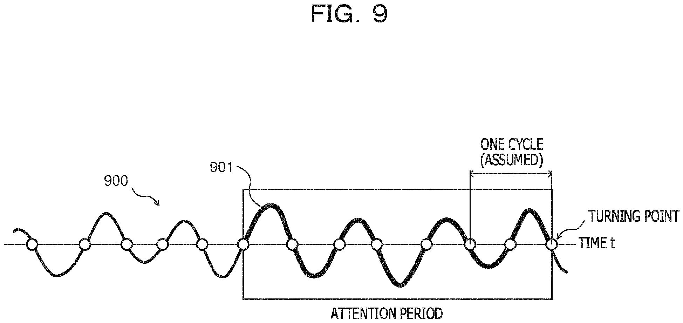

Accordingly, the information processing apparatus 100 can generate the pulse wave waveforms at any part of the subject who is the determination target of whether or not to indicate the arrhythmia. Further, the information processing apparatus 100 can generate the pulse wave waveforms of each of the parts of the subject, which is used for calculating the first index value. Here, the description of FIG. 9 will be made.

<One Example of Setting Attention Period>

FIG. 9 is an explanatory view illustrating an example of setting an attention period. In FIG. 9, the information processing apparatus 100 specifies a turning point at which the waveform changes from positive to negative or from negative to positive in a generated pulse wave waveform 900 at any part of the subject. Next, for example, the information processing apparatus 100 sets a period having a length that corresponds to a predetermined number of cycles as an attention period, assuming that the length of three turning points is one cycle. The predetermined number of cycles may not be an integral multiple period. The predetermined number of cycles is, for example, 3.5 cycles. The predetermined number of cycles may be, for example, four cycles or the like. The information processing apparatus 100 may set a plurality of attention periods by setting the attention period for each period having the length that corresponds to the predetermined number of cycles.

In addition, the information processing apparatus 100 divides a partial waveform 901 within the attention period in the generated pulse wave waveform 900 at any part of the subject into the partial waveform 901 of the determination target for determining whether or not the noise is included. The information processing apparatus 100 may set a plurality of attention periods for the moving image 101 photographed within the predetermined period. In addition, the information processing apparatus 100 may set one or more plural attention periods for each of the moving images 101 when there are a plurality of moving images 101 photographed within the predetermined period. The information processing apparatus 100 sets the partial waveform 901 within each of the attention periods as the partial waveform 901 that serves as a determination target of whether or not to indicate an arrhythmia. In addition, in a case where a plurality of attention periods are set, the attention periods may partially overlap each other. Here, the description of FIG. 10 will be made.

<One Example of Calculating First Index Value>

FIG. 10 is an explanatory view illustrating a first example of calculating the first index value. Here, the information processing apparatus 100 uses a value indicating the matching degree between the pulse wave waveforms of the pulse wave waveforms of each of the parts that correspond to the change in volume of the blood that flows through the blood vessel at each of the parts of the subject, as the first index value.

When there is no the body movement of the subject, the pulse wave waveforms of each of the parts tend to become a waveform that corresponds to the blood flow similarly flowing from the heart due to expansion and contraction of the heart, and thus, the matching degree tends to increase. Therefore, the first index value indicating the matching degree indicates that the body movement of the subject increases as the value decreases and the pulse wave waveform is in a state of being likely to be disturbed by the noise caused by the body movement of the subject, and indicates the likelihood of the pulse wave is small.

In FIG. 10, the information processing apparatus 100 calculates a value indicating the matching degree between the partial waveforms within the attention period in the pulse wave waveforms of each of the parts, as the first index value. For example, the information processing apparatus 100 extracts a partial waveform 1011 within the attention period in a pulse wave waveform 1010 on the right side of the face. In addition, the information processing apparatus 100 acquires a data string x(i) of the extracted partial waveform 1011. x(i) is a data string in which the values x(1), ... , x(X) of the partial waveform 1011 within the attention period are arranged in the order of time. For example, the unit of i is millisecond.

Further, the information processing apparatus 100 extracts a partial waveform 1021 within another period in which the attention period of the pulse wave waveform on the left side of the face is shifted by a shift width z. In addition, the information processing apparatus 100 acquires a data string y(z, i) of the extracted partial waveform 1021. y(z, i) is a data string in which the values y(z, 1), ... , x(z, X) of the partial waveform 1021 within other periods shifted by the shift width z from the attention period are arranged in the order of time. For z = 0, y(z, i) is the partial waveform values y(z, 1), ... , y(z, X) within the attention period. For example, the unit of z is millisecond.

In addition, the information processing apparatus 100 sets z = 0 and substitutes the data string x(i) and the data string y(z, i) into the following equation (1). Accordingly, the information processing apparatus 100 calculates a correlation coefficient s(z) as the first index value indicating the matching degree between the partial waveform 1011 within the attention period on the right side of the face and the partial waveform 1021 within the attention period on the left side of the face.

[Equation 1]

$$s(z) = \frac{\sum_{i=1}^{x}(x(i)-\bar{x})(y(z,i)-\overline{y(z)})}{\sqrt{\sum_{i=1}^{x}(x(i)-\bar{x})^2}\sqrt{\sum_{i=1}^{x}(y(z,i)-\overline{y(z)})^2}} \quad \cdots (1)$$

$\bar{x}$ is an average of x(1), ..., x(X). $\overline{y(z)}$ is an average of y(z, 1), ..., y(z, X).

Here, a case where the information processing apparatus 100 calculates the first index value indicating the matching degree between the partial waveform 1011 within the attention period on the right side of the face and the partial waveform 1021 within the attention period on the left side of the face by using the above-described equation (1) has been described, but the present invention is not limited thereto. For example, the information processing apparatus 100 may calculate the correlation coefficient s(z) of the following equation (2) as the first index value indicating the matching degree between the partial waveforms within the attention period between the partial waveforms on the right side of the face and on the left side of the face by substituting the data string x(i) and the data string y(z, i) into the following equation (2).

[Equation 2]

$$S(z) = \frac{\sum_{i=1}^{x}(x(i)-\bar{x})(y(z,i)-\overline{y(z)})}{\max\left(\sum_{i=1}^{x}(x(i)-\bar{x})^2, \sum_{i=1}^{x}(y(z,i)-\overline{y(z)})^2\right)} \quad \cdots (2)$$

Further, for example, the information processing apparatus 100 may calculate the first index value indicating the matching degree between the partial waveform 1011 within the attention period on the right side of the face and the partial waveform 1021 within the attention period on the left side of the face by using a Euclidean distance or the normalized Euclidean distance.

In addition, here, a case where the information processing apparatus 100 calculates the first index value indicating the matching degree based on the pulse wave waveforms of each of the parts of the two parts has been described, but the present invention is not limited thereto. For example, the information processing apparatus 100 may calculate the first index value based on three or more pulse wave waveforms.

Specifically, in a case where there are pulse wave waveforms of each of the parts of the forehead, the left cheek, and the right cheek, the information processing apparatus 100 may acquire the data string indicating the partial waveform within the attention period of each of the parts. Next, the information processing apparatus 100 calculates the correlation coefficient for the combination of each of the data strings by substituting the combination of each of the data strings into the above-described equation (1). In addition, the information processing apparatus 100 calculates statistical values, such as an average value, a median value, a maximum value, and a minimum value of the calculated correlation coefficients as the first index value. Accordingly, the information processing apparatus 100 can improve the likelihood of the first index value in a case where each of the parts is not on the same plane.

Specifically, the information processing apparatus 100 may calculate the correlation coefficient for each of the corresponding combinations, such as a combination of the left cheek and the right cheek or a combination of the upper part of the face and the lower part of the face in a case where there are pulse wave waveforms of each of the parts, such as the left cheek, the right cheek, the upper part of the face, and the lower part of the face. In addition, the information processing apparatus 100 calculates statistical values, such as an average value, a median value, a maximum value, and a minimum value of the calculated correlation coefficients as the first index value. Accordingly, the information processing apparatus 100 can calculate the first index value that can correspond to various body movements of the subject, and can improve the likelihood of the first index value.

Here, a case where the information processing apparatus 100 calculates the first index value for one attention period has been described, but the present invention is not limited thereto. For example, in a case where a plurality of attention periods are set, the information processing apparatus 100 calculates a first index value for each of the attention periods. Here, the description of FIG. 11 will be made.

FIG. 11 is an explanatory view illustrating a second example of calculating the first index value. Here, in a case where each of the parts of the subject are physically separated from each other, since the distance from the heart is different, the pulse wave waveforms of each of the parts have similar waveforms, but there is a case where the waveforms are shifted in a time direction. Here, when calculating the first index value, the information processing apparatus 100 may calculate the first index value indicating the matching degree of the pulse wave waveforms at any part of the subject and the pulse wave waveform at another part.

In FIG. 11, the information processing apparatus 100 calculates the first index value indicating the matching degree of the partial waveform within the attention period of the pulse wave waveforms at any part and the partial waveform within another period shifted from the attention period by the shift width z in the pulse wave waveform at another part.

For example, the information processing apparatus 100 extracts the partial waveform 1011 within the attention period in the pulse wave waveform 1010 on the right side of the face, and acquires the data string x(i) of the extracted partial waveform 1011. x(i) is a data string in which the values x(1), ... , x(X) of the partial waveform 1011 within the attention period are arranged in the order of time. For example, the unit of i is millisecond.

In addition, while incrementing the range of z = -100 to 100 by 1, the information processing apparatus 100 extracts a partial waveform 1100 within another period shifted from the attention period in a pulse wave waveform 1020 on the left side of the face by the shift width z. In addition, the information processing apparatus 100 acquires the data string y(z, i) of the extracted partial waveform 1100. y(z, i) is a data string in which the values y(z, 1), ... , x(z, X) of the partial waveform 1100 within other periods shifted by the shift width z from the attention period are arranged in the order of time. For example, the unit of z is millisecond.

In addition, for example, while incrementing the interval of z = -100 to 100 one by one, the information processing apparatus 100 calculates the correlation coefficient s(z) by substituting the data string x(i) and the data string y(z, i) in the above-described equation (1) or the equation (2). Further, the information processing apparatus 100 sets the maximum value among the correlation coefficients s(z) of z = -100 to 100 as the first index value.

Here, a case where the information processing apparatus 100 calculates the first index value by using the above-described equation (1) or the equation (2) has been described, but the present invention is not limited thereto. For example, the information processing apparatus 100 may calculate the first index value by using the Euclidean distance or the normalized Euclidean distance.

In addition, here, a case where the information processing apparatus 100 calculates the first index value based on the pulse wave waveforms of each of the parts of the two parts has been described, but the present invention is not limited thereto. For example, the information processing apparatus 100 may calculate the first index value based on the pulse wave waveforms of each of the parts of the three or more parts.

Specifically, in a case where there are pulse wave waveforms of each of the parts of the forehead, the left cheek, and the right cheek, the information processing apparatus 100 calculates the correlation coefficient of the partial waveform within the attention period and the partial waveform within another period shifted from the attention period by the shift width z for each of the combinations of the parts. In addition, the information processing apparatus 100 may calculate statistical values, such as an average value, a median value, a maximum value, and a minimum value of the calculated correlation coefficients as the first index value. Accordingly, the information processing apparatus 100 can improve the likelihood of the first index value in a case where each of the parts is not on the same plane.

More specifically, the information processing apparatus 100 may calculate the correlation coefficient for each of the corresponding combinations, such as a combination of the left cheek and the right cheek or a combination of the upper part of the face and the lower part of the face in a case where there are pulse wave waveforms of each of the parts, such as the left cheek, the right cheek, the upper part of the face, and the lower part of the face. In addition, the information processing apparatus 100 calculates the minimum value of the calculated correlation coefficients as the first index value. The information processing apparatus 100 may calculate the statistical values, such as an average value, a median value, and a maximum value of the calculated correlation coefficients as the first index value. Accordingly, the information processing apparatus 100 can calculate the first index value that can correspond to various body movements of the subject, and can improve the likelihood of the first index value. Here, the description of FIG. 12 will be made.

<One Example of Calculating Second Index Value>

FIG. 12 is an explanatory view illustrating an example of calculating the second index value. As a technology for calculating the second index value, for example, the technology described in Japanese Laid-open Patent Publication No. 2014-176584 can be referred to.

In FIG. 12, the information processing apparatus 100 calculates the second index value indicating the matching degree of the partial waveform within the attention period and the partial waveform within another period shifted from the attention period by the shift width z in the pulse wave waveforms at any part of the subject. For example, the information processing apparatus 100 extracts the partial waveform 901 within the attention period in the pulse wave waveform 900 of the face. In addition, the information processing apparatus 100 acquires the data string x(i) of the extracted partial waveform 901. x(i) is a data string in which the values x(1), ... , x(X) of the partial waveform 901 within the attention period are arranged in the order of time. For example, the unit of i is millisecond.

In addition, while incrementing the range of z = 400 to 1500 by 1, the information processing apparatus 100 extracts a partial waveform 1200 within another period shifted from the attention period in the pulse wave waveform 900 of the face by the shift width z. In addition, the information processing apparatus 100 acquires the data string y(z, i) of the extracted partial waveform 1200. y(z, i) is a data string in which the values y(z, 1), ... , x(z, X) of the partial waveform 1200 within other periods shifted by the shift width z from the attention period are arranged in the order of time. For example, the unit of z is millisecond.

Next, while incrementing the interval of z = -400 to 1500 one by one, the information processing apparatus 100 calculates the correlation coefficient s(z) by substituting the data string x(i) and the data string y(z, i) in the above-described equation (1) or the equation (2). Further, the information processing apparatus 100 sets the maximum value among the correlation coefficients s(z) of z = 400 to 1500 as the second index value. Further, the information processing apparatus 100 may adopt z when the correlation coefficient s(z) becomes the maximum among z = 400 to 1500 as the pulse wave interval.

Here, a case where the information processing apparatus 100 calculates the second index value indicating the matching degree by using the above-described equation (1) or the equation (2) has been described, but the present invention is not limited thereto. For example, the information processing apparatus 100 may calculate the second index value indicating the matching degree by using the Euclidean distance or the normalized Euclidean distance.

Here, a case where the information processing apparatus 100 calculates the second index value for one attention period has been described, but the present invention is not limited thereto. For example, in a case where a plurality of attention periods are set, the information processing apparatus 100 calculates the second index value for each of the attention periods. Here, the description of FIG. 13 will be made.

<One Example of Determining whether or not Pulse Wave Waveform indicates Arrhythmia by First Determination Pattern>

FIG. 13 is an explanatory view illustrating an example of determining whether or not the partial waveform within the attention period indicates an arrhythmia according to the first determination pattern. Here, the first index value indicates that the possibility of disturbance of the pulse wave waveform by the noise caused by the body movement of the subject in the pulse wave waveforms at any part of the subject is high as the value decreases.

Meanwhile, in a case where the value is large, the second index value indicates that the pulse wave waveform is periodic. In other words, the second index value tends to be a waveform in which the pulse wave waveform indicates a normal pulse as the value increases. Therefore, the information processing apparatus 100 can determine whether or not the pulse wave waveforms indicate an arrhythmia, based on the tendency of the first index value and the tendency of the second index value.

In FIG. 13, in a case where the first index value is equal to or greater than the first threshold value, the information processing apparatus 100 determines that the noise is not included in the partial waveform within the attention period of the pulse wave waveforms at any part of the subject. The first threshold value is, for example, 0.6. Furthermore, in a case where the second index value is equal to or greater than the fifth threshold value, the information processing apparatus 100 may determine that the noise is not included in the partial waveform within the attention period of the pulse wave waveforms at any part of the subject. The fifth threshold value is, for example, 0.7.

In addition, in a case where it is determined that the noise is not included, when the second index value is less than the second threshold value, the information processing apparatus 100 determines that the partial waveform within the attention period in the pulse wave waveforms at any part of the subject indicate an arrhythmia. The second threshold value is, for example, 0.5. In addition, in a case where it is determined that the noise is not included, when the second index value is equal to or greater than the second threshold value, the information processing apparatus 100 may determine that the partial waveform within the attention period in the pulse wave waveforms at any part of the subject indicate an arrhythmia.

In a case where the average value of the first index value is calculated, the information processing apparatus 100 may determine whether or not the partial waveform within the attention period includes the noise by comparing the average value of the first index values and the first threshold value to each other. In addition, in a case where the minimum value of the first index value is calculated, the information processing apparatus 100 may determine whether or not the partial waveform within the attention period includes the noise by comparing the minimum value of the first index values and the first threshold value to each other. Here, the description of FIG. 14 will be made.

FIG. 14 is an explanatory view illustrating an example of determining whether or not the pulse wave waveform within the predetermined period indicates the arrhythmia according to the first determination pattern. In the example of FIG. 14, by using a case where a plurality of attention periods are set within the predetermined period as an example, one example in which it is determined whether or not the pulse wave waveform within the predetermined period indicates an arrhythmia by performing the statistical processing for the plurality of attention periods, will be described.

In FIG. 14, in a case where the calculated first index value is equal to or greater than the first threshold value for each of the attention periods, the information processing apparatus 100 determines that the noise is not included in the partial waveform within the attention period in the pulse wave waveforms at any part of the subject. Furthermore, in a case where the calculated second index value is equal to or greater than the fifth threshold value for each of the attention periods, the information processing apparatus 100 may determine that the noise is not included in the partial waveform within the attention period in the pulse wave waveforms at any part of the subject.

Next, the information processing apparatus 100 determines whether or not the second index value calculated for each of the attention periods during which it is determined that the noise is not included is less than the second threshold value. In addition, when the ratio of the number of attention periods during which it is determined that the second index value is less than the second threshold value with respect to the number of attention periods during which it is determined that the noise is not included, is equal to or greater than a sixth threshold value, the information processing apparatus 100 determines that the pulse wave waveforms at any part of the subject within the predetermined period indicate an arrhythmia. The sixth threshold value is, for example, 0.05. In addition, when the ratio of the number of attention periods during which it is determined that the second index value is less than the second threshold value with respect to the number of attention periods during which it is determined that the noise is not included, is less than the sixth threshold value, the information processing apparatus 100 may determine that the pulse wave waveforms at any part of the subject within the predetermined period indicate a normal pulse.

In addition, in a case where the number of attention periods during which it is determined that the noise is not included with respect to the set number of attention periods is equal to or less than a seventh threshold value, the information processing apparatus 100 may determine that the pulse wave waveforms at any part of the subject within the predetermined period indicate an arrhythmia. The seventh index value is, for example, 0.5.

Accordingly, the information processing apparatus 100 can determine whether or not the pulse wave waveforms at any part of the subject within the predetermined period statistically indicate an arrhythmia, based on the first index value and the second index value which are calculated for each of the attention periods within the predetermined period. Therefore, the information processing apparatus 100 can improve the determination accuracy for determining whether or not the pulse wave waveforms at any part of the subject indicates an arrhythmia. Here, the description of FIG. 15 will be made.

<One Example of Determining whether or not Pulse Wave Waveform indicates Arrhythmia by Second Determination Pattern>

FIG. 15 is an explanatory view illustrating an example of determining whether or not the pulse wave waveform within the predetermined period indicates the arrhythmia according to the second determination pattern. Here, according to the arrhythmia, the pulse wave waveforms of each of the parts of the subject has a similar disturbance pattern, and the pulse wave waveforms at any part of the subject tend to be not periodic. In other words, according to the arrhythmia, while the first index value indicating the matching degree between the pulse wave waveforms of each of the parts of the subject remains high, the second index value indicating the matching degree between the partial waveforms in the pulse wave waveforms at any part of the subject becomes lower.

Meanwhile, when the noise is generated with respect to the normal pulse, the pulse wave waveforms of each of the parts of the subject has a similar disturbance pattern, and the pulse wave waveforms at any part of the subject tend to be not periodic. In other words, according to the noise, the first index value indicating the matching degree between the pulse wave waveforms of each of the parts of the subject remains high, and the second index value indicating the matching degree between the partial waveforms in the pulse wave waveforms at any part of the subject becomes lower.

As described above, according to the arrhythmia, the second index value tends to increase or decrease regardless of the first index value, and the intensity of the correlation between the first index value and the second index value tends to relatively become smaller. In addition, according to noise, the first index value and the second index value tend to become smaller together, and the intensity of the correlation between the first index value and the second index value tends to become relatively larger. Here, the information processing apparatus 100 determines whether or not the pulse wave waveforms at any part of the subject indicate an arrhythmia, based on the value indicating the intensity of the correlation between the first index value and the second index value.

In FIG. 15, by using the correlation coefficient, as the third index value, the information processing apparatus 100 calculates the value indicating the intensity of the correlation between the first index value and the second index value, based on the first index value and the second index value for each of the calculated attention periods. As the third index value, by substituting the combination of the first index value and the second index value for each of the calculated attention periods into the calculation formula of the correlation coefficient, the information processing apparatus 100 calculates the value of the correlation coefficient indicating the intensity of the correlation between the first index value and the second index value.

In addition, in a case where the third index value is equal to or less than the third threshold value, the information processing apparatus 100 determines that the pulse wave waveforms at any part of the subject within the predetermined period indicate the arrhythmia. Further, in a case where the third index value is greater than the third threshold value, the information processing apparatus 100 may determine that the pulse wave waveforms at any part of the subject within the predetermined period indicate a normal pulse.

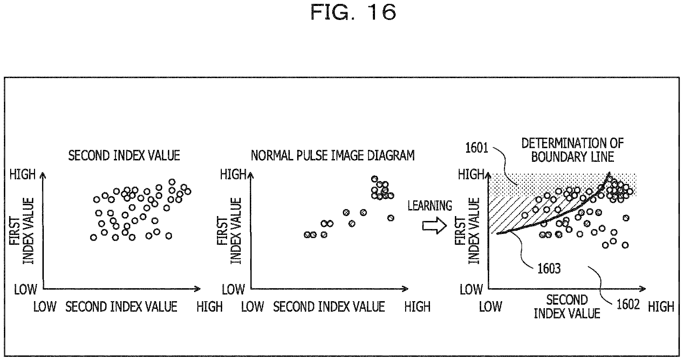

Accordingly, even when the noise is included in the pulse wave waveforms at any part of the subject within the predetermined period, the information processing apparatus 100 can determine whether or not the pulse wave waveforms at any part of the subject within the predetermined period indicates an arrhythmia. In addition, the information processing apparatus 100 can determine whether or not the pulse wave waveforms at any part of the subject within the predetermined period statistically indicate an arrhythmia, based on the first index value and the second index value which are calculated for each of the attention periods within the predetermined period. Therefore, the information processing apparatus 100 can improve the determination accuracy for determining whether or not the pulse wave waveforms at any part of the subject indicates an arrhythmia. Here, the description of FIG. 16 will be made.

FIG. 16 is an explanatory view illustrating an example of determining whether or not the partial waveform within the attention period indicates an arrhythmia according to the second determination pattern. Here, there is a tendency that a range that can be taken by the combination of the first index value and the second index value in a case of an arrhythmia and a range that can be taken by the combination of the first index value and the second index value in a case of a normal pulse are different from each other.

For example, in the two-dimensional space based on the axis of the first index value and the axis of the second index value, there is a region 1601 where the combination of the first index value and the second index value appears in a case of an arrhythmia. In addition, in the two-dimensional space based on the axis of the first index value and the axis of the second index value, there is a region 1602 where the combinations of the first index value and the second index value exist together in a case of a normal pulse and in a case of an arrhythmia. A boundary line 1603 can be set between the region 1601 and the region 1602.

Here, the information processing apparatus 100 sets in advance a model indicating the boundary line 1603 that can specify the region 1601 where the combination of the first index value and the second index value appears in a case of an arrhythmia, and determines whether or not the partial waveform within the attention period indicates an arrhythmia based on the model. In the following description, there is a case where the region 1601 where the combination of the first index value and the second index value in a case of an arrhythmia appears is referred to as "arrhythmia region 1601". In addition, there is a case where the region 1602 where the combinations of the first index value and the second index value in a case of the normal pulse or in a case of the arrhythmia exist together and appears is referred to as a "region 1602 where the normal pulse and the arrhythmia exist together".

In FIG. 16, the information processing apparatus 100 determines whether or not the combination of the first index value and the second index value calculated for the attention period is included in the arrhythmia region 1601 specified by the preset model. In addition, in a case where it is determined that the combination is included in the arrhythmia region 1601, the information processing apparatus 100 determines that the partial waveform within the attention period indicates an arrhythmia.

The model is, for example, an equation illustrating the boundary line 1603 between the arrhythmia region 1601 and the region 1602 where the normal pulse and the arrhythmia exist together. The equation is set, for example, by a learning process that uses learning data, such as the combination or the like of the first index value and the second index value obtained from the pulse wave waveform determined to indicate the normal pulse or the arrhythmia in advance.

Specifically, the information processing apparatus 100 determines whether or not the first index value calculated for the attention period is equal to or greater than the first index value that corresponds to the second index value calculated for the attention period specified by the equation. In addition, in a case where the calculated first index value is equal to or greater than the first index value that corresponds to the calculated second index value, the information processing apparatus 100 determines that the partial waveform within the attention period in the pulse wave waveforms at any part of the subject indicate an arrhythmia.

In addition, by using a support vector machine and the like, the information processing apparatus 100 may determine whether or not the combination of the first index value and the second index value calculated for the attention period is included in the arrhythmia region 1601. Accordingly, even when the noise is included in the partial waveform within the attention period in the pulse wave waveforms at any part of the subject, the information processing apparatus 100 can determine whether or not the partial waveforms within the attention period indicates an arrhythmia.

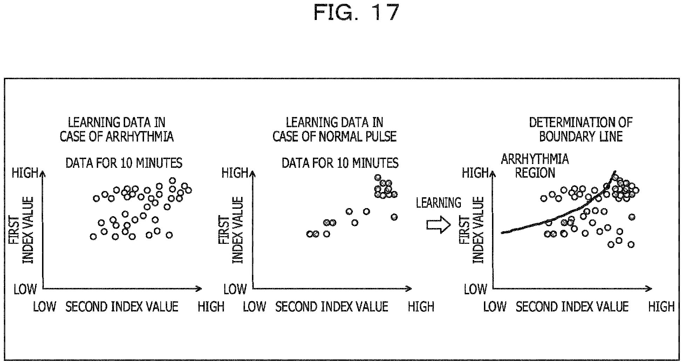

The information processing apparatus 100 may further determine whether or not the noise is included in the partial waveform within the attention period in a case where it is determined that the partial waveform does not indicate the arrhythmia after determining whether or not the partial waveform within the attention period in the pulse wave waveforms at any part of the subject indicates an arrhythmia. Here, the description of FIG. 17 will be made.

FIG. 17 is an explanatory view illustrating an example of generating the model for specifying the arrhythmia region 1601. Here, in a case where no learning data is obtained, the information processing apparatus 100 can adopt the combination of the first index value and the second index value for a predetermined period during which it is determined whether or not to indicate an arrhythmia by the processes of FIGs. 13 and 14, as the learning data.

In FIG. 17, the information processing apparatus 100 performs the processes of FIGs. 13 and 14 for a certain predetermined period and determines whether or not to indicate an arrhythmia with respect to the partial waveforms within each of the attention periods included in the predetermined period. Next, in a case where it is determined that the arrhythmia is not indicated for the partial waveforms within each of the attention periods, the information processing apparatus 100 acquires the combination of the first index value and the second index value calculated for each of the attention periods.

In addition, the information processing apparatus 100 adopts the acquired combination as the learning data of the combination of the first index value and the second index value in a case of a normal pulse. In the following description, there is a case where the learning data of the combination of the first index value and the second index value in a case of a normal pulse is referred to as "learning data in a case of a normal pulse".

In addition, the information processing apparatus 100 performs the processes of FIGs. 13 and 14 for a certain predetermined period and determines whether or not to indicate an arrhythmia with respect to the pulse wave waveform within the predetermined period. Further, in a case where it is determined that the pulse wave waveform within the predetermined period indicates an arrhythmia, the information processing apparatus 100 acquires the combination of the first index value and the second index value calculated for each of the attention periods within the predetermined period.

In addition, the information processing apparatus 100 adopts the acquired combination as the learning data of the combination of the first index value and the second index value in a case of an arrhythmia. In the following description, there is a case where the learning data of the combination of the first index value and the second index value in a case of an arrhythmia is referred to as "learning data in a case of an arrhythmia".

The information processing apparatus 100 generates a model that specifies the arrhythmia region 1601 based on the acquired learning data. The information processing apparatus 100 generates, for example, an equation illustrating the boundary line 1603 between the arrhythmia region 1601 and the region 1602 where the normal pulse and the arrhythmia exist together. Accordingly, the information processing apparatus 100 can generate and set the model even when the learning data may not be obtained in advance. In addition, the information processing apparatus 100 can perform the process of FIG. 16 based on the generated model.

In addition, the information processing apparatus 100 can generate the model based on the learning data on the subject that becomes a generation target which generates the pulse wave waveform that serves as a determination target for determining whether or not to indicate an arrhythmia in the future. Therefore, when determining whether or not the pulse wave waveform generated for the subject in the future indicates an arrhythmia, the information processing apparatus 100 can use the model with a high possibility of being suitable for the subject. In addition, the information processing apparatus 100 can improve the determination accuracy for determining whether or not the pulse wave waveforms of the subject indicates an arrhythmia.

<One Example of Analyzing Pulse Wave Waveform according to Determination Result>

The information processing apparatus 100 analyzes the pulse wave waveforms at any part of the subject by using the partial waveform determined that the noise in the pulse wave waveforms at any part of the subject is not included. In a case where the partial waveform determined to include no noise and the partial waveform determined to include the noise overlap each other, the information processing apparatus 100 may use a part that does not overlap the partial waveform determined to include the noise in the partial waveform determined to include no noise. Accordingly, the information processing apparatus 100 can analyze the pulse wave waveforms at any part of the subject without using the partial waveform including the noise, and it is possible to improve the analysis accuracy.

For example, the information processing apparatus 100 calculates the pulse rate [bpm] by analyzing the pulse wave waveforms at any part of the subject. Further, the information processing apparatus 100 may analyze the pulse wave waveforms at any part of the subject, differentiate the pulse wave waveforms at any part of the subject twice, calculate an acceleration pulse wave waveform, and calculate a vascular age from the acceleration pulse wave waveform. Further, for example, the information processing apparatus 100 may analyze the pulse wave waveforms at any part of the subject and determine whether or not the subject has an arrhythmia.

The information processing apparatus 100 may analyze the pulse wave waveforms at any part of the subject who is determined to exhibit an arrhythmia. For example, the information processing apparatus 100 may analyze at which moment the symptom of the arrhythmia appears in the subject, from which time the pulse wave waveforms at any part of the subject determined to indicate an arrhythmia is measured. In addition, the information processing apparatus 100 may analyze how frequently the symptoms of the arrhythmia appear to the subject based on the number of pulse wave waveforms at any part of the subject who is determined to exhibit an arrhythmia within a period, such as one month.

Here, a case where the information processing apparatus 100 analyzes the pulse wave waveforms at any part of the subject without using the partial waveform including the noise, has been described, but the invention is not limited thereto. For example, the information processing apparatus 100 may analyze the pulse wave waveforms at any part of the subject by using the first index value, the second index value, and the pulse wave waveforms at any part of the subject. In addition, the information processing apparatus 100 may notify the user of the first index value, the second index value, and the pulse wave waveforms at any part of the subject, and support the user to analyze the pulse wave waveforms at any part of the subject.

(One Example of Overall Processing Procedure)

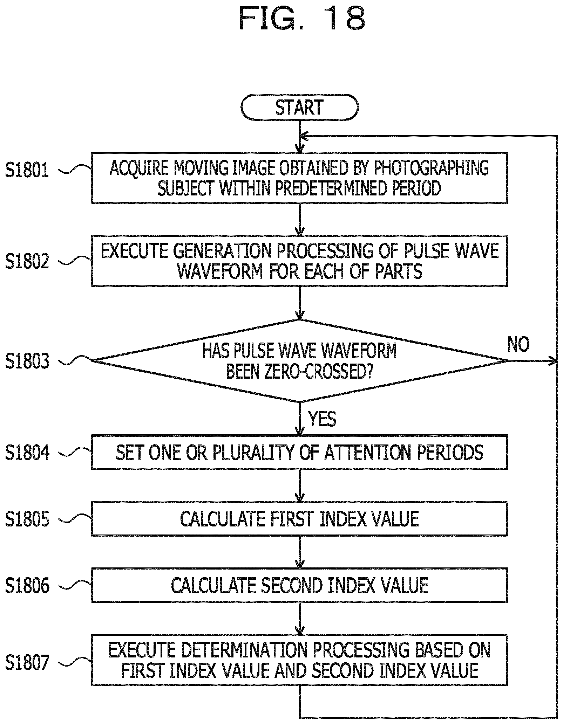

Next, an example of the overall processing procedure executed by the information processing apparatus 100 will be described with reference to FIG. 18. In the example of FIG. 18, the information processing apparatus 100 adopts the predetermined entire region, the left region, and the right region as a plurality of parts. The predetermined entire region is, for example, the face of the subject. The left region is a part included on the left side of the entire region, for example, the left cheek. The right region is a part included on the right side of the entire region, for example, the right cheek.

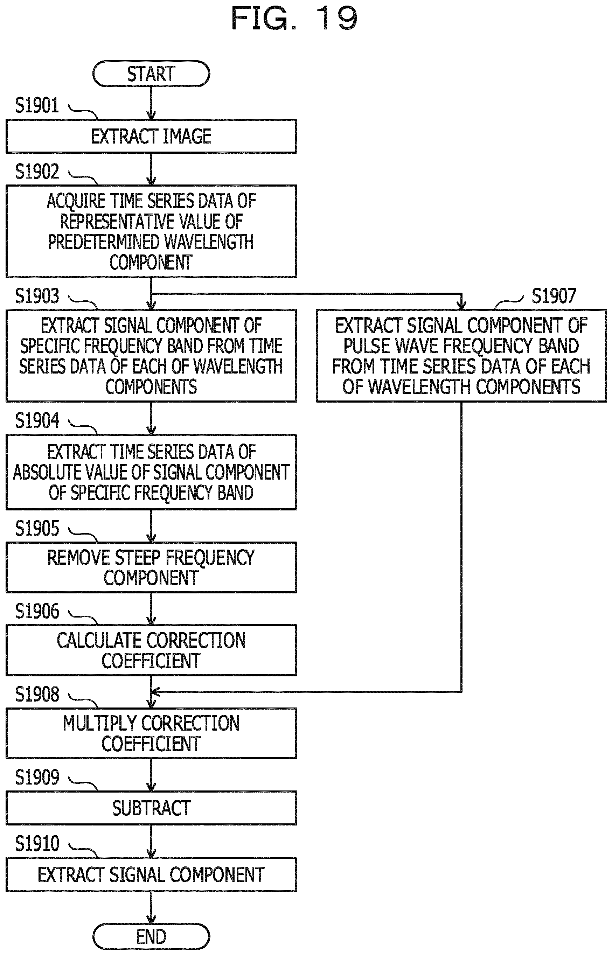

FIG. 18 is a flowchart illustrating an example of the overall processing procedure. In FIG. 18, the information processing apparatus 100 acquires the moving image 101 obtained by photographing the subject within the predetermined period (step S1801). Next, the information processing apparatus 100 executes generation processing of the pulse wave waveform (to be described later in FIG. 19) for each of the parts of the plurality of parts of the subject (step S1802).

In addition, the information processing apparatus 100 determines whether or not the pulse wave waveform of the generated entire region has zero-crossed (step S1803). The zero crossing means that the pulse wave waveform includes a turning point at which the amplitude changes from positive to negative or from negative to positive. Here, in a case where the zero-crossing is not performed (step S1803: No), the information processing apparatus 100 returns to the process of step S1801. Meanwhile, in s case of zero-crossing (step S1803: Yes), the information processing apparatus 100 sets one or a plurality of attention periods based on the generated pulse wave waveform of the entire region (step S1804).

Next, for each of the set attention periods, the information processing apparatus 100 calculates the first index value indicating the matching degree between the partial waveforms within the attention period of the generated pulse wave waveforms of the left region and the right region (step S1805). In addition, for each of the set attention periods, the information processing apparatus 100 calculates the second index value indicating the matching degree between the partial waveforms of the generated pulse wave waveforms of the entire region (step S1806).

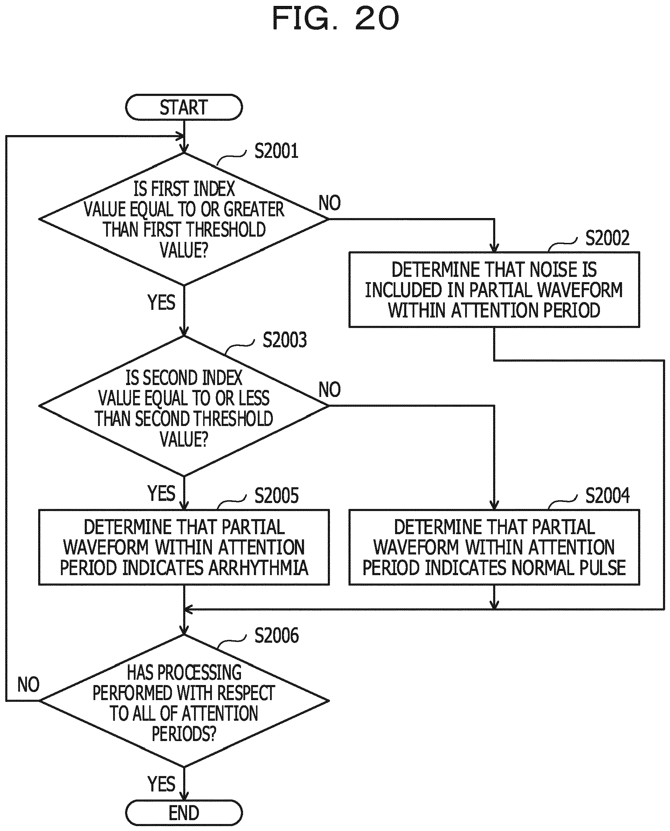

Next, based on the calculated first index value and second index value, the information processing apparatus 100 performs any determination processing which will be described later with reference to FIGs. 20 to 25 for the generated pulse wave waveform of the entire region (step S1807). In addition, the information processing apparatus 100 returns to the process of step S1801. Accordingly, the information processing apparatus 100 can determine whether or not the pulse wave waveform in the entire region of the person indicates an arrhythmia, and can improve the analysis accuracy of the pulse wave waveform in the entire region of the person.

(One Example of Generation Processing Procedure)

Next, an example of the generation processing procedure executed by the information processing apparatus 100 will be described with reference to FIG. 19.

FIG. 19 is a flowchart illustrating an example of the generation processing procedure. In FIG. 19, the information processing apparatus 100 extracts the images of each of the parts of the subject from the moving image 101 (step S1901). Next, based on the extracted image, the information processing apparatus 100 acquires the time series data of the representative values of predetermined wavelength components included in the image (step S1902). In addition, the information processing apparatus 100 extracts the signal component in the specific frequency band from the time series data for each of the acquired wavelength components by the BPF (step S1903).

Next, the information processing apparatus 100 extracts the time series data of the absolute value of the signal component in the specific frequency band from the signal component in the specific frequency band for each of the extracted wavelength components (step S1904). In addition, the information processing apparatus 100 performs the smoothing process with respect to the time series data of the absolute value of the specific frequency band for each of the extracted wavelength components by the LPF, and removes steep frequency components (step S1905).

Next, the information processing apparatus 100 calculates a correction coefficient based on the time series data of the absolute value in the specific frequency band for each of the smoothed wavelength components (step S1906). In addition, together with steps S1903 to S1906, the information processing apparatus 100 extracts the signal component in the pulse wave frequency band from the time series data for each of the acquired wavelength components by the BPF (step S1907).

Next, in the information processing apparatus 100, the signal component of any of the extracted pulse wave frequency bands is multiplied by the calculated correction coefficient (step S1908). In addition, in the information processing apparatus 100, the signal component of the pulse wave frequency band of the wavelength component multiplied by a correction coefficient k is subtracted from the signal component of the pulse wave frequency band of any wavelength component (step S1909).

Next, the information processing apparatus 100 extracts the signal component of the pulse wave frequency band of the time series data of the signal after the subtraction by the BPF (step S1910). In addition, the information processing apparatus 100 ends the generation processing. Accordingly, the information processing apparatus 100 can generate the pulse wave waveforms of each of the parts of the subject.

(One Example of Determination Processing Procedure)

Next, one example of the determination processing procedure executed by the information processing apparatus 100 will be described with reference to FIGs. 20 to 25.

< First Example of Determination Processing Procedure>

FIG. 20 is a flowchart illustrating a first example of the determination processing procedure. The determination processing in FIG. 20 corresponds to the processing in FIG. 13, for example.

In FIG. 20, the information processing apparatus 100 determines whether or not the first index value calculated for any attention period is equal to or greater than the first threshold value (step S2001). Here, in a case of being less than the first threshold value (step S2001: No), the information processing apparatus 100 determines that noise is included in the partial waveform within the attention period (step S2002), and moves to the process of step S2006.

Meanwhile, in a case of being equal to or greater than the first threshold value (step S2001: Yes), the information processing apparatus 100 determines whether or not the second index value calculated for the attention period is equal to or less than the second threshold value (step S2003). Here, in a case of being greater than the second threshold value (step S2003: No), the information processing apparatus 100 determines that the partial waveform within the attention period indicates a normal pulse (step S2004) and moves to the process of step S2006.

Meanwhile, in a case of being equal to or less than the second threshold value (step S2003: Yes), the information processing apparatus 100 determines that the partial waveform within the attention period indicates an arrhythmia (step S2005) and moves to the process of step S2006. In step S2006, the information processing apparatus 100 determines whether or not the process has been performed for all of the attention periods (step S2006).

Here, in a case where there is the attention period during which the process is not performed (step S2006: No), the information processing apparatus 100 returns to the process of step S2001. Meanwhile, in a case where the process has been performed for all of the attention periods (step S2006: Yes), the information processing apparatus 100 ends the determination processing. Accordingly, the information processing apparatus 100 can determine whether or not the partial waveform within the attention period of the pulse wave waveform of the entire region of the subject indicates an arrhythmia.

<Second Example of Determination Processing Procedure>

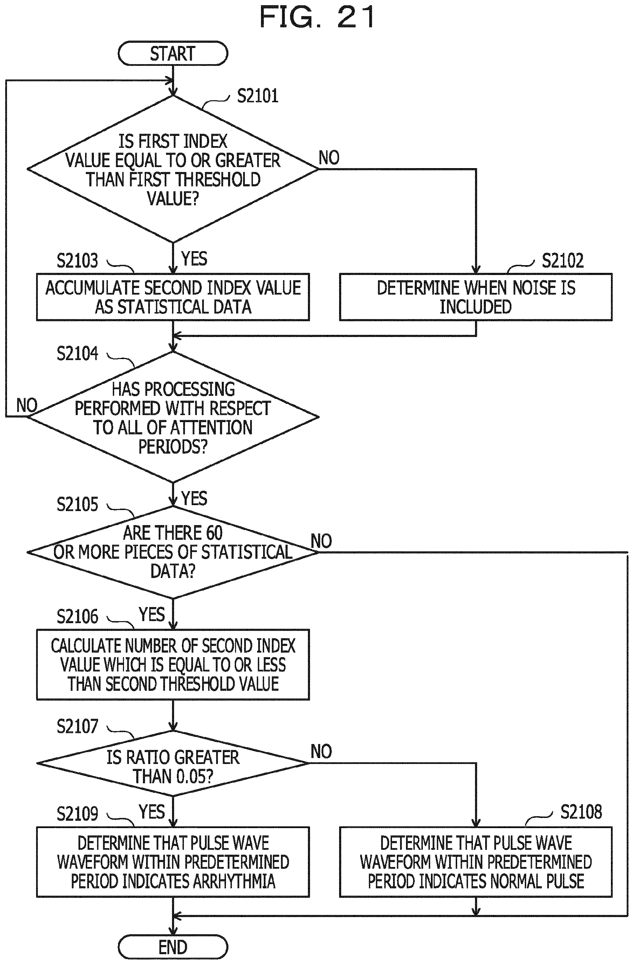

FIG. 21 is a flowchart illustrating a second example of the determination processing procedure. FIG. 21 corresponds to the process of FIG. 14, for example.

In FIG. 21, the information processing apparatus 100 determines whether or not the first index value calculated for any attention period is equal to or greater than the first threshold value (step S2101). Here, in a case of being less than the first threshold value (step S2101: No), the information processing apparatus 100 determines that noise is included in the partial waveform within the attention period (step S2102), and moves to the process of step S2104.

Meanwhile, in a case of being equal to or greater than the first threshold value (step S2101: Yes), the information processing apparatus 100 accumulates the second index value calculated for the attention period as statistical data (step S2103). In addition, the information processing apparatus 100 moves to the process of step S2104.

In step S2104, the information processing apparatus 100 determines whether or not the process has been performed for all of the attention periods (step S2104). Here, in a case where there is the attention period during which the process is not performed (step S2104: No), the information processing apparatus 100 returns to the process of step S2101.

Meanwhile, in a case where the process has been performed for all of the attention periods (step S2104: Yes), the information processing apparatus 100 determines whether or not there are 60 or more pieces of statistical data (step S2105). Here, in a case where there are not 60 or more pieces of statistical data (step S2105: No), the information processing apparatus 100 ends the determination processing.

Meanwhile, in a case where there are 60 or more pieces of statistical data (step S2105: Yes), the information processing apparatus 100 calculates the second index value which is equal to or less than the second threshold value among the second index values accumulated as the statistical data (step S2106). Next, the information processing apparatus 100 determines whether or not the ratio obtained by dividing the calculated number by the number of pieces of statistical data is greater than 0.05 (step S2107). Here, in a case where the number of pieces of statistical data is less than 0.05 (step S2107: No), the information processing apparatus 100 determines that the pulse wave waveform within the predetermined period indicates a normal pulse (step S2108) and ends the determination processing.

Meanwhile, in a case where the number of pieces of statistical data is greater than 0.05 (step S2107: Yes), the information processing apparatus 100 determines that the pulse wave waveform within the predetermined period indicates an arrhythmia (step S2109) and ends the determination processing. Accordingly, the information processing apparatus 100 can determine whether or not the pulse wave waveform of the entire region of the subject indicates an arrhythmia.

<Third Example of Determination Processing Procedure>

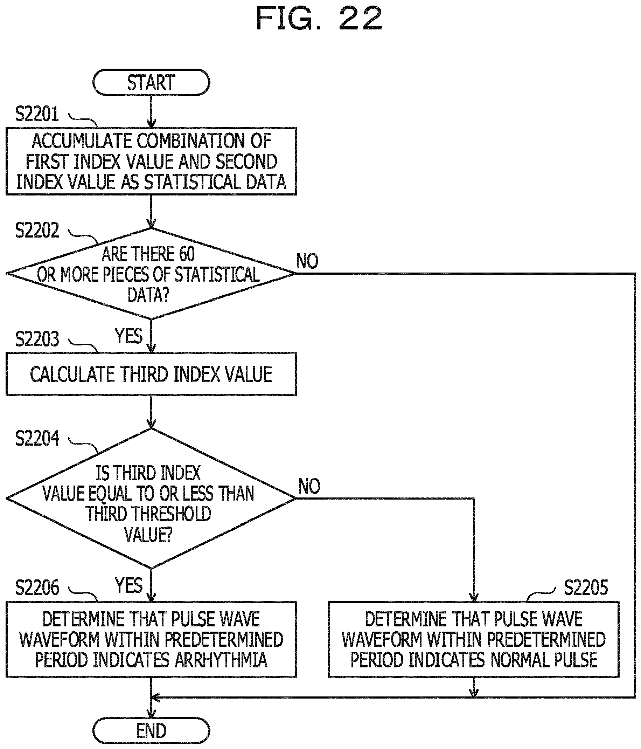

FIG. 22 is a flowchart illustrating a third example of the determination processing procedure. FIG. 22 corresponds to the process of FIG. 15, for example. In FIG. 22, the information processing apparatus 100 accumulates the combination of the first index value and the second index value calculated for each of the attention periods as the statistical data (step S2201). Next, the information processing apparatus 100 determines whether or not there are 60 or more pieces of statistical data (step S2202). Here, in a case where there are not 60 or more pieces of statistical data (step S2202: No), the information processing apparatus 100 ends the determination processing.

Meanwhile, in a case where there are 60 or more pieces of statistical data (step S2202: Yes), the information processing apparatus 100 calculates the third index value which indicates the intensity of the correlation between the first index value and the second index value based on the combination accumulated as the statistical data (step S2203). Next, the information processing apparatus 100 determines whether or not the calculated third index value is equal to or less than the third threshold value (step S2204).

Here, in a case where the third index value is greater than the third threshold value (step S2204: No), the information processing apparatus 100 determines that the pulse wave waveform within the predetermined period indicates a normal pulse (step S2205) and ends the determination processing. Meanwhile, in a case where the third index value is equal to or less than the third threshold value (step S2204: Yes), the information processing apparatus 100 determines that the pulse wave waveform within the predetermined period indicates an arrhythmia (step S2206) and ends the determination processing. Accordingly, the information processing apparatus 100 can determine whether or not the pulse wave waveform of the entire region of the subject indicates an arrhythmia.

< Fourth Example of Determination Processing Procedure>

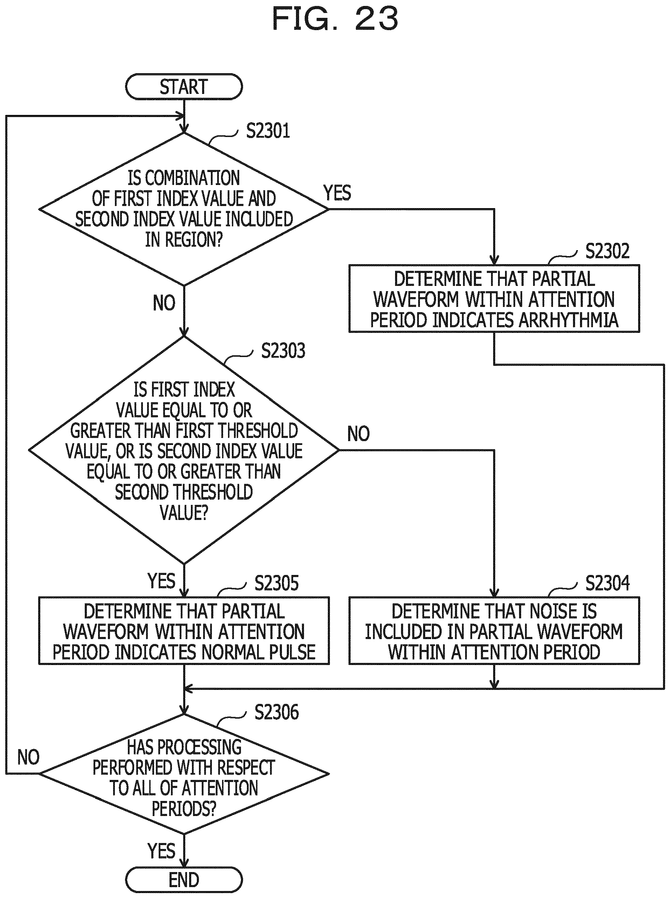

FIG. 23 is a flowchart illustrating a fourth example of the determination processing procedure. FIG. 23 corresponds to the process of FIG. 16, for example.

In FIG. 23, the information processing apparatus 100 determines whether or not the combination of the first index value and the second index value is included in the region where the combination between the first index value and the second index value in a case of an arrhythmia specified by the model appears (step S2301). Here, in a case where the combination is included in the region (step S2301: Yes), the information processing apparatus 100 determines that the partial waveform within the attention period indicates an arrhythmia (step S2302) and moves to the process of step S2306.

Meanwhile, in a case where the combination is not included in the region (step S2301: No), the information processing apparatus 100 determines whether the first index value is equal to or greater than the first threshold value or the second index value is equal to or greater than the second threshold value (step S2303). Here, in a case where the first index value is smaller than the first threshold value or the second index value is smaller than the second threshold value (step S2303: No), the information processing apparatus 100 determines that the noise is included in the partial waveform within the attention period (step S2304), and moves to the process of step S2306.

Meanwhile, in a case where the first index value is equal to or greater than the first threshold value and the second index value is equal to or greater than the second threshold value (step S2303: Yes), the information processing apparatus 100 determines that the partial waveform within the attention period indicates a normal pulse (step S2305) and moves to the process of step S2306. In step S2306, the information processing apparatus 100 determines whether or not the process has been performed for all of the attention periods (step S2306).

Here, in a case where there is the attention period during which the process is not performed (step S2306: No), the information processing apparatus 100 returns to the process of step S2301. Meanwhile, in a case where the process has been performed for all of the attention periods (step S2306: Yes), the information processing apparatus 100 ends the determination processing. Accordingly, the information processing apparatus 100 can determine whether or not the partial waveform within the attention period of the pulse wave waveform of the entire region of the subject indicates an arrhythmia.

< Fifth Example of Determination Processing Procedure>

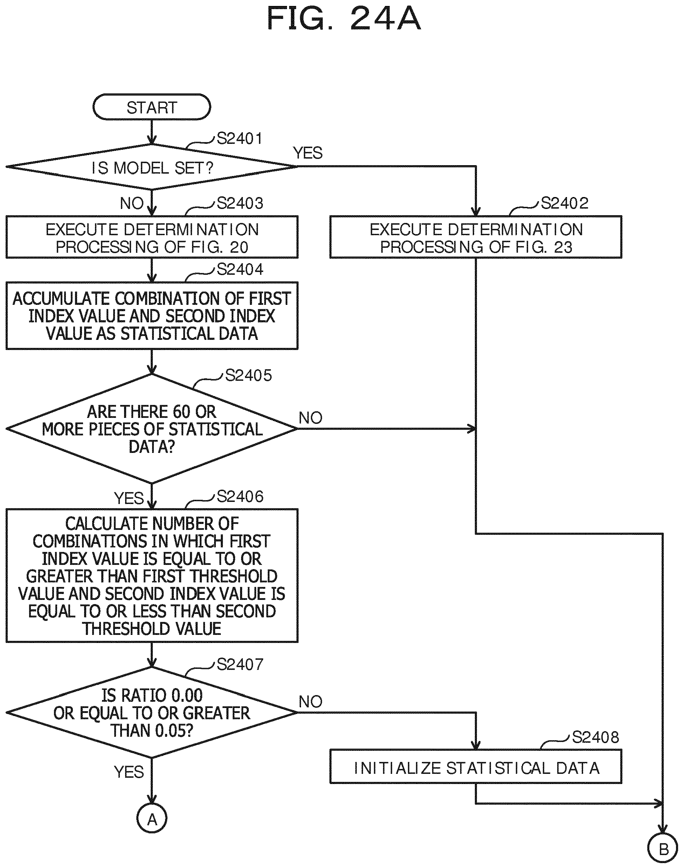

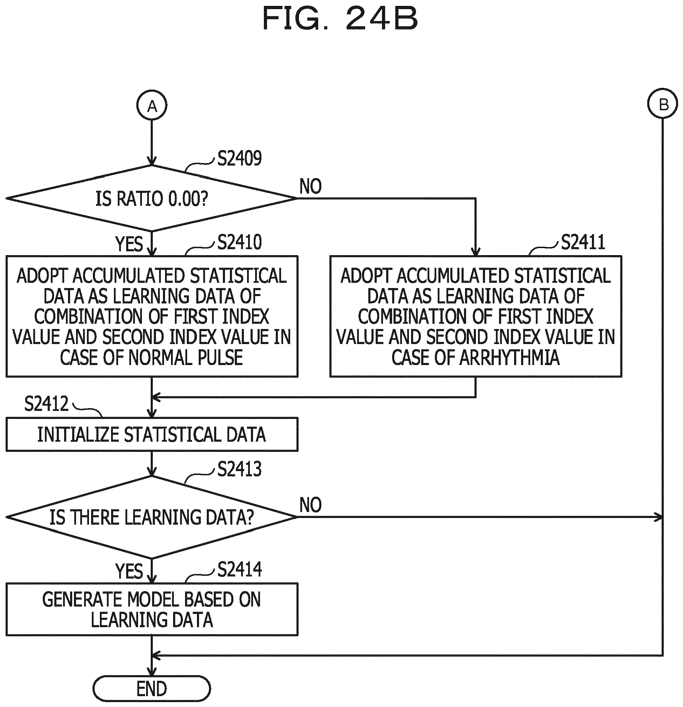

FIG. 24 is a flowchart illustrating a fifth example of the determination processing procedure. FIG. 24 corresponds to the process of FIG. 17, for example.

In FIG. 24, the information processing apparatus 100 determines whether or not the model is set (step S2401). Here, in a case where the model is set (step S2401: Yes), the information processing apparatus 100 executes the determination processing of FIG. 23 (step S2402) and ends the determination processing of FIG. 24.

Meanwhile, in a case where the model is not set (step S2401: No), the information processing apparatus 100 executes the determination processing of FIG. 20 (step S2403), and determines whether or not the partial waveform within the attention period indicates an arrhythmia. Next, the information processing apparatus 100 accumulates the combination of the first index value and the second index value calculated for the attention period that is the determination result that does not include the noise in the attention period as the statistical data (step S2404). Next, the information processing apparatus 100 determines whether or not there are 60 or more pieces of statistical data (step S2405). Here, in a case where there are not 60 or more pieces of statistical data (step S2405: No), the information processing apparatus 100 ends the determination processing.

Meanwhile, in a case where there are 60 or more pieces of statistical data (step S2405: Yes), the information processing apparatus 100 calculates the number of combinations in which the first index value is equal to or greater than the first threshold value and the second index value is equal to or less than the second threshold value among the accumulated combinations (step S2406). Next, the information processing apparatus 100 determines whether the ratio obtained by dividing the calculated number by the number of pieces of statistical data is 0.00 or equal to or greater than 0.05 (step S2407).

Here, in a case where the number of pieces of statistical data is not 0.00 or smaller than 0.05 (step S2407: No), the information processing apparatus 100 initializes the statistical data (step S2408) and ends the determination processing. Meanwhile, in a case where the number of pieces of statistical data is 0.00 or equal to or greater than 0.05 (step S2407: Yes), the information processing apparatus 100 divides the calculated number by the number of pieces of statistical data and determines whether or not the obtained ratio is 0.00 (step S2409).

Here, in a case where the number of pieces of statistical data is 0.00 (step S2409: Yes), the information processing apparatus 100 adopts the accumulated statistical data as the learning data of the combination of the first index value and the second index value in a case of a normal pulse (step S2410). In addition, the information processing apparatus 100 moves to the process of step S2412.

Meanwhile, in a case where the number of pieces of statistical data is not 0.00 (step S2409: No), the information processing apparatus 100 adopts the accumulated statistical data as the learning data of the combination of the first index value and the second index value in a case of an arrhythmia (step S2411). In addition, the information processing apparatus 100 moves to the process of step S2412.

In step S2412, the information processing apparatus 100 initializes the statistical data (step S2412). In addition, the information processing apparatus 100 determines whether or not there is the learning data of the combination of the first index value and the second index value in a case of a normal pulse and in a case of an arrhythmia used for generating the model (step S2413). Here, in a case where there is no learning data (step S2413: No), the information processing apparatus 100 ends the determination processing.

Meanwhile, in a case where there is the learning data (step S2413: Yes), the information processing apparatus 100 generates the model based on the learning data (step S2414) and ends the determination processing. Accordingly, the information processing apparatus 100 can determine whether or not the partial waveform within the attention period of the pulse wave waveform of the entire region of the subject indicates an arrhythmia, based on the model.

Further, in a case where the model is not set, the information processing apparatus 100 can generate the model from the combination of the first index value and the second index value calculated for the subject. Therefore, the information processing apparatus 100 can generate the model that corresponds to the subject, and can make it easy to determine whether or not the partial waveform within the attention period of the pulse wave waveforms at any part of the subject indicates an arrhythmia based on the model with high accuracy.

<Sixth Example of Determination Processing Procedure>

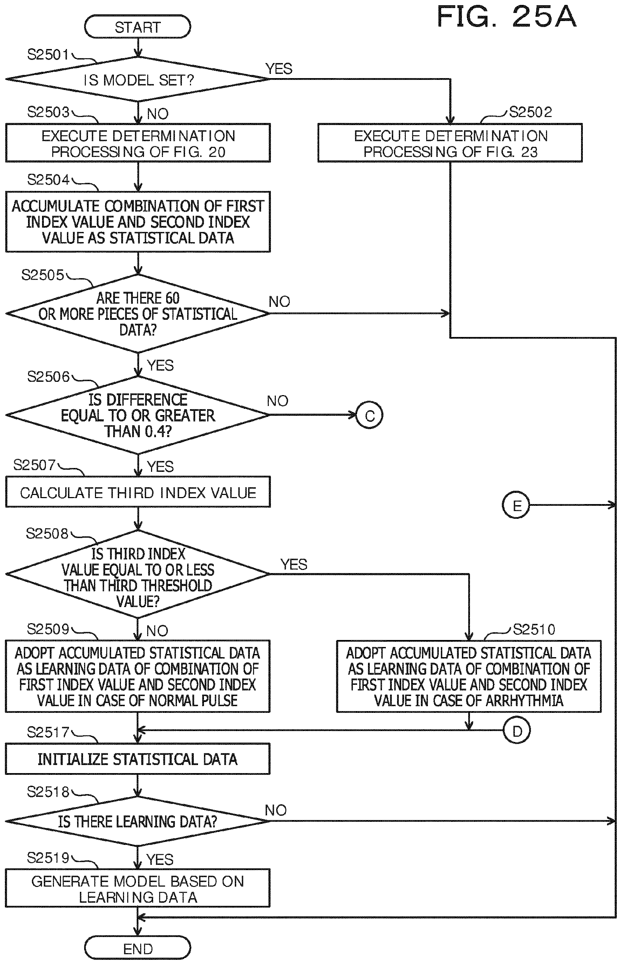

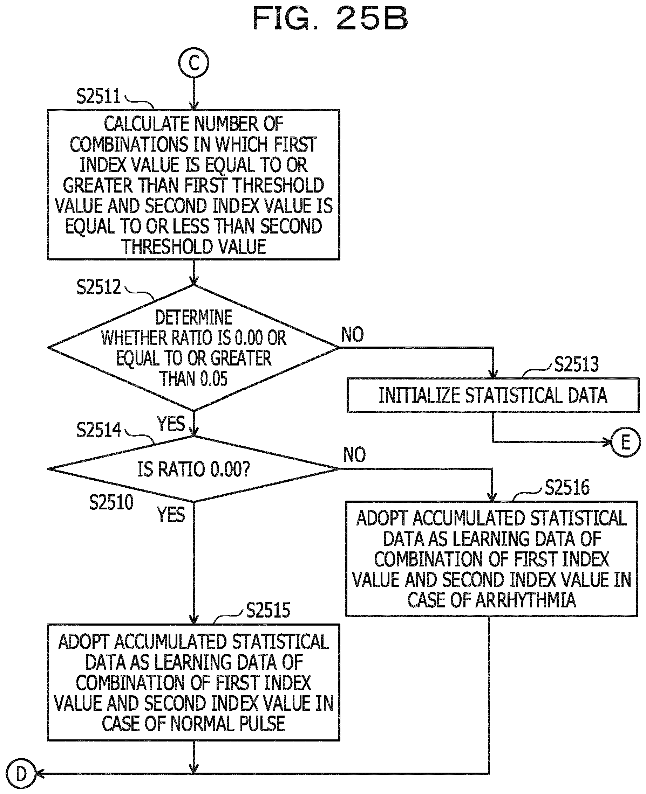

FIG. 25 is a flowchart illustrating a sixth example of the determination processing procedure. In FIG. 25, the information processing apparatus 100 determines whether or not the model is set (step S2501). Here, in a case where the model is set (step S2501: Yes), the information processing apparatus 100 executes the determination processing of FIG. 23 (step S2502) and ends the determination processing of FIG. 25.

Meanwhile, in a case where the model is not set (step S2501: No), the information processing apparatus 100 executes the determination processing of FIG. 20 (step S2503), and determines whether or not the partial waveform within the attention period indicates an arrhythmia. Next, the information processing apparatus 100 accumulates the combination of the first index value and the second index value calculated for the attention period that is the determination result that does not include the noise in the attention period as the statistical data (step S2504). Next, the information processing apparatus 100 determines whether or not there are 60 or more pieces of statistical data (step S2505). Here, in a case where there are not 60 or more pieces of statistical data (step S2505: No), the information processing apparatus 100 ends the determination processing.

Meanwhile, in a case where there are 60 or more pieces of statistical data (step S2505: Yes), the information processing apparatus 100 determines whether or not a difference when the minimum value of the second index value is subtracted from the maximum value of the second index value is equal to or greater than 0.4 based on the statistical data (step S2506). Here, in a case where the difference is equal to or greater than 0.4 (step S2506: Yes), the information processing apparatus 100 calculates the third index value which indicates the intensity of the correlation between the first index value and the second index value based on the combination accumulated as the statistical data (step S2507).

Next, the information processing apparatus 100 determines whether or not the calculated third index value is equal to or less than the third threshold value (step S2508). Here, in a case where the third index value is greater than the third threshold value (step S2508: No), the information processing apparatus 100 adopts the accumulated statistical data as the learning data of the combination of the first index value and the second index value in a case of a normal pulse (step S2509). In addition, the information processing apparatus 100 moves to the process of step S2517.

Meanwhile, in a case where the third index value is equal to or less than the third threshold value (step S2508: Yes), the information processing apparatus 100 adopts the accumulated statistical data as the learning data of the combination of the first index value and the second index value in a case of an arrhythmia (step S2510). In addition, the information processing apparatus 100 moves to the process of step S2517.

In addition, in step S2506, in a case where the difference is smaller than 0.4 (step S2506: No), the information processing apparatus 100 calculates the number of combinations in which the first index value is equal to or greater than the first threshold value and the second index value is equal to or less than the second threshold value among the accumulated combinations (step S2511). Next, the information processing apparatus 100 determines whether the ratio obtained by dividing the calculated number by the number of pieces of statistical data is 0.00 or equal to or greater than 0.05 (step S2512).

Here, in a case where the number of pieces of statistical data is not 0.00 or smaller than 0.05 (step S2512: No), the information processing apparatus 100 initializes the statistical data (step S2513) and ends the determination processing. Meanwhile, in a case where the number of pieces of statistical data is 0.00 or equal to or greater than 0.05 (step S2512: Yes), the information processing apparatus 100 divides the calculated number by the number of pieces of statistical data and determines whether or not the obtained ratio is 0.00 (step S2514).

Here, in a case where the number of pieces of statistical data is 0.00 (step S2514: Yes), the information processing apparatus 100 adopts the accumulated statistical data as the learning data of the combination of the first index value and the second index value in a case of a normal pulse (step S2515). In addition, the information processing apparatus 100 moves to the process of step S2517.

Meanwhile, in a case where the number of pieces of statistical data is not 0.00 (step S2514: No), the information processing apparatus 100 adopts the accumulated statistical data as the learning data of the combination of the first index value and the second index value in a case of an arrhythmia (step S2516). In addition, the information processing apparatus 100 moves to the process of step S2517.

In step S2517, the information processing apparatus 100 initializes the statistical data (step S2517). In addition, the information processing apparatus 100 determines whether or not there is the learning data of the combination of the first index value and the second index value in a case of a normal pulse and in a case of an arrhythmia used for generating the model (step S2518). Here, in a case where there is no learning data (step S2518: No), the information processing apparatus 100 ends the determination processing.

Meanwhile, in a case where there is the learning data (step S2518: Yes), the information processing apparatus 100 generates the model based on the learning data (step S2519) and ends the determination processing. Accordingly, the information processing apparatus 100 can determine whether or not the partial waveform within the attention period of the pulse wave waveform of the entire region of the subject indicates an arrhythmia, based on the model.

Further, in a case where the model is not set, the information processing apparatus 100 can generate the model from the combination of the first index value and the second index value calculated for the subject. Therefore, the information processing apparatus 100 can generate the model that corresponds to the subject, and can make it easy to determine whether or not the partial waveform within the attention period of the pulse wave waveforms at any part of the subject indicates an arrhythmia based on the model with high accuracy.

As described above, according to the information processing apparatus 100, it is possible to calculate the first index value indicating the matching degree between at least any one of the pulse wave waveforms of the pulse wave waveforms of each of the parts of the subject. In addition, according to the information processing apparatus 100, it is possible to calculate the second index value indicating the matching degree between the partial waveforms in the pulse wave waveforms at any part of the subject. In addition, according to the information processing apparatus 100, it is possible to determine whether or not the pulse wave waveforms at any part of the subject indicate an arrhythmia based on the combination of the calculated first index value and the second index value, and the information indicating the correspondence relationship of the first index value and the second index value in a case of an arrhythmia.

Accordingly, the information processing apparatus 100 can refer to the likelihood of the pulse wave of the pulse wave waveforms at any part of the subject and the matching degree between the partial waveforms of the pulse wave waveforms at any part of the subject, and can determine whether or not the pulse wave waveforms at any part of the subject indicates an arrhythmia. As a result, the information processing apparatus 100 can improve the analysis accuracy of the pulse wave waveforms at any part of the subject with reference to the determined result.

In addition, according to the information processing apparatus 100, in a case where it is determined that the calculated first index value is equal to or greater than the first index value that corresponds to the calculated second index value, it is possible to determine that the pulse wave waveforms at any part of the subject indicate an arrhythmia. Accordingly, even when it is determined whether or not the noise is included in the pulse wave waveforms at any part of the subject, the information processing apparatus 100 can determine whether or not the pulse wave waveforms at any part of the subject within the predetermined period indicate an arrhythmia.

In addition, according to the information processing apparatus 100, it is possible to calculate the first index value indicating the matching degree between at least any one of the pulse wave waveforms of the pulse wave waveforms of each of the parts of the subject for each of the periods within the predetermined period. In addition, according to the information processing apparatus 100, it is possible to calculate the second index value indicating the matching degree between the partial waveforms of the pulse wave waveforms of at least one of the parts for each of the periods within the predetermined period. Further, according to the information processing apparatus 100, it is possible to calculate the third index value indicating the intensity of the correlation between the first index value and the second index value based on the first index value and the second index value calculated for each of the periods. In addition, according to the information processing apparatus 100, in a case where it is determined that the calculated third index value is equal to or less than the threshold value, it is possible to determine that the pulse wave waveforms at any part within the predetermined period indicate an arrhythmia. Accordingly, even when it is determined whether or not the noise is included in the pulse wave waveforms at any part of the subject, the information processing apparatus 100 can determine whether or not the pulse wave waveforms at any part of the subject within the predetermined period indicate an arrhythmia.

In addition, according to the information processing apparatus 100, based on the first index value calculated for each of the periods within the predetermined period, it is possible to determine whether or not the noise is included in the pulse wave waveforms at any part in each of the periods. In addition, according to the information processing apparatus 100, based on the second index value calculated for each of the periods during which it is determined that the noise is not included within the predetermined period, it is possible to determine whether or not the pulse wave waveforms at any part for each of the periods during which it is determined that the noise is not included indicate an arrhythmia. In addition, according to the information processing apparatus 100, based on the determination result as to whether or not the pulse wave waveforms at any part in each of the periods during which it is determined that the noise is not included indicate an arrhythmia, it is possible to generate information indicating the correspondence relationship between the first index value and the second index value in a case of an arrhythmia.

Accordingly, even when the information indicating the correspondence relationship used when determining whether or not the pulse wave waveforms at any part of the subject within the predetermined period indicates an arrhythmia, is set in advance, the information processing apparatus 100 can generate the information indicating the correspondence relationship. Further, since the information processing apparatus 100 generates the information indicating the correspondence relationships for the subject, the information processing apparatus 100 can improve the determination accuracy of whether or not to indicate the arrhythmia with respect to the pulse wave waveform of the subject.

Further, according to the information processing apparatus 100, based on the second index value calculated for each of the periods, it is possible to calculate the fourth index value indicating the magnitude of the variation of the second index value. In addition, according to the information processing apparatus 100, in a case where the fourth index value is equal to or less than the threshold value, based on the first index value calculated for each of the periods within the predetermined period, it is possible to determine whether or not the noise is included in the pulse wave waveforms at any part in each of the periods. In addition, according to the information processing apparatus 100, based on the second index value calculated for each of the periods during which it is determined that the noise is not included within the predetermined period, it is possible to determine whether or not the pulse wave waveforms at any part for each of the periods during which it is determined that the noise is not included indicate an arrhythmia.

Accordingly, in a case where the disturbance of the pulse wave waveforms at any part of the subject is relatively small, the information processing apparatus 100 determines whether or not the noise is included in the pulse wave waveforms at any part of the subject, and in a case where the noise is not included in the pulse wave waveform, it is possible to determine whether or not to indicate an arrhythmia. As a result, the information processing apparatus 100 excludes the pulse wave waveform including the noise from an analysis target, can analyze the pulse wave waveform that does not include the noise as an analysis target by referring to the determination result as to whether or not to indicate an arrhythmia, and can improve the analysis accuracy.

In addition, according to the information processing apparatus 100, it is possible to determine whether or not the second index value calculated for each of the periods during which it is determined that the noise is not included within the predetermined period is smaller than the second threshold value. In addition, according to the information processing apparatus 100, based on the ratio of the period during which it is determined that the second index value is smaller than the second threshold value in the period during which it is determined that the noise is not included within the predetermined period, it is possible to determine whether or not the pulse wave waveforms at any part within the predetermined period indicates an arrhythmia. Accordingly, by using the second index value calculated only in a period during which the noise is not included, the information processing apparatus 100 can determine whether or not the pulse wave waveforms at any part of the subject within the predetermined period indicates an arrhythmia and can improve the determination accuracy.

Here, in a case where the disturbance of the pulse wave waveforms at any part of the subject is relatively large, when the pulse wave waveform that does not include the noise is regarded as the analysis target without regarding the pulse wave waveform including the noise as the analysis target, there is a possibility that the number of pulse wave waveforms that are the analysis target becomes smaller and the analysis accuracy deteriorates. Here, according to the information processing apparatus 100, in a case where the fourth index value is greater than the fourth threshold value, it is possible to calculate the third index value indicating the intensity of the correlation between the first index value and the second index value based on the first index value and the second index value calculated for each of the periods. In addition, according to the information processing apparatus 100, in a case where it is determined that the calculated third index value is equal to or less than the third threshold value, it is possible to determine that the pulse wave waveforms at any part within the predetermined period indicate an arrhythmia.

Accordingly, in a case where the disturbance of the pulse wave waveforms at any part of the subject is relatively large, the information processing apparatus 100 determines whether or not the pulse wave waveform indicates an arrhythmia without determining whether or not the noise is included in the pulse wave waveforms at any part of the subject. In addition, the information processing apparatus 100 can analyze the pulse wave waveform as an analysis target regardless of whether or not the noise is not included by referring to the result of determining whether or not the pulse wave waveform indicates an arrhythmia regardless of whether or not the noise is included, and can improve the analysis accuracy.

Further, according to the information processing apparatus 100, it is possible to calculate the first index value indicating the matching degree between at least any one of the pulse wave waveforms of the pulse wave waveforms of each of the parts of the subject. In addition, according to the information processing apparatus 100, it is possible to calculate the second index value indicating the matching degree between the partial waveforms of the pulse wave waveforms at any part of the pulse wave waveforms of each of the parts of the subject. In addition, according to the information processing apparatus 100, based on the calculated first index value, it is possible to determine whether or not the noise is included in the pulse wave waveforms at any part of the subject. In addition, according to the information processing apparatus 100, it is possible to determine whether or not the pulse wave waveforms at any part of the subject indicate an arrhythmia based on the calculated second index value in a case where it is determined that the noise is not included.

Accordingly, the information processing apparatus 100 determines whether or not the noise is included in the pulse wave waveforms at any part of the subject, and in a case where the noise is not included in the pulse wave waveform, it is possible to determine whether or not to indicate an arrhythmia. As a result, the information processing apparatus 100 excludes the pulse wave waveform including the noise from the analysis target, can analyze the pulse wave waveform that does not include the noise as an analysis target by referring to the determination result as to whether or not to indicate an arrhythmia, and can improve the analysis accuracy.

In addition, according to the information processing apparatus 100, it is possible to calculate the first index value indicating the matching degree between the pulse wave waveforms of the acquired pulse wave waveforms of each of the parts of the subject for each of the periods within the predetermined period. In addition, according to the information processing apparatus 100, it is possible to calculate the second index value indicating the matching degree between the partial waveforms of the acquired pulse wave waveforms at any part for each of the periods within the predetermined period. In addition, according to the information processing apparatus 100, based on the first index value calculated for each of the periods within the predetermined period, it is possible to determine whether or not the noise is included in the pulse wave waveforms at any part in each of the periods. In addition, according to the information processing apparatus 100, it is possible to determine whether or not the second index value calculated for each of the periods during which it is determined that the noise is not included within the predetermined period is greater than the second threshold value. In addition, according to the information processing apparatus 100, based on the ratio of the period during which it is determined that the second index value is greater than the second threshold value in the period during which it is determined that the noise is not included within the predetermined period, it is possible to determine whether or not the pulse wave waveforms at any part within the predetermined period indicates an arrhythmia.

Accordingly, without using the second index value calculated for the period during which the noise is included, the information processing apparatus 100 can determine whether or not the pulse wave waveforms at any part of the subject within the predetermined period indicates an arrhythmia and can improve the determination accuracy.

In addition, according to the information processing apparatus 100, based on the first index value and the calculated second index value, it is possible to determine whether or not the noise is included in the pulse wave waveforms at any part. Accordingly, the information processing apparatus 100 can refer to the likelihood of the pulse wave of the pulse wave waveforms at any part of the subject and the matching degree between the partial waveforms of the pulse wave waveforms at any part of the subject, can determine whether or not the noise is included, and can improve the determination accuracy.

In addition, according to the information processing apparatus 100, it is possible to adopt the value indicating the matching degree between the partial waveforms having the length that corresponds to the predetermined cycle in the pulse wave waveforms at any part, as the second index value. Accordingly, the information processing apparatus 100 can adopt the value indicating how similar partial waveforms are repeated for the pulse wave waveforms at any part of the subject, as the second index value.

In addition, according to the information processing apparatus 100, it is possible to adopt the value indicating the magnitude of the variation in the pulse wave intervals among the pulse wave waveforms at any part, as the second index value. Accordingly, the information processing apparatus 100 can adopt the value indicating the magnitude of change in cycle with respect to the pulse wave waveforms at any part of the subject, as the second index value.

In addition, the information processing method described in the embodiment can be realized by executing a prepared program on a computer, such as a personal computer or a workstation. The information processing program is recorded in a recording medium that can be read by the computer, such as a hard disk, a flexible disk, a CD-ROM, an MO, or a DVD, and is executed by being read from the recording medium by the computer. Also, the information processing program may be distributed via a network, such as the Internet.

Reference Signs List

```
100 INFORMATION PROCESSING APPARATUS
101 MOVING IMAGE
200 BUS
201 CPU
202 MEMORY
203 I/F
204 DISK DRIVE
205 DISK
206 CAMERA
210 NETWORK
301 ACQUIRING UNIT
302 CALCULATION UNIT
303 DETERMINATION UNIT
304 ANALYSIS UNIT
305 OUTPUT UNIT
400 PHOTOGRAPHED IMAGE
410 to 412, 501 to 503, 601, 602 IMAGES
801,805,808 BPF
802, 804, 806, 807 OPERATION UNIT
803 LPF
900, 1010, 1020 PULSE WAVE WAVEFORM
901, 1011, 1021, 1100, 1200 PARTIAL WAVEFORM
1601, 1602 REGION
1603 BOUNDARY LINE
```

All examples and conditional language recited herein are intended for pedagogical purposes to aid the reader in understanding the invention and the concepts contributed by the inventor to furthering the art, and are to be construed as being without limitation to such specifically recited examples and conditions, nor does the organization of such examples in the specification relate to a showing of the superiority and inferiority of the invention. Although the embodiments of the present invention have been described in detail, it should be understood that the various changes, substitutions, and alterations could be made hereto without departing from the spirit and scope of the invention.

**Claims**

1. An information processing apparatus comprising:

an acquiring unit that acquires pulse wave waveforms of each of a plurality of parts obtained by analyzing images of each of the parts of a subject included in each photographed image obtained by photographing the subject;
a calculation unit that calculates a first index value indicating a matching degree between the pulse wave waveforms of the acquired pulse wave waveforms of each of the parts, and calculates a second index value indicating a matching degree between partial waveforms of the acquired pulse wave waveforms of each of the parts;
a determination unit that determines whether or not the acquired pulse wave waveform indicates an arrhythmia based on a combination of the calculated first index value and the calculated second index value; and
an output unit that outputs a determination result as to whether or not to indicate the arrhythmia.

2. The information processing apparatus according to claim 1,
wherein the determination unit determines whether or not the pulse wave waveform indicates the arrhythmia based on information indicating a correspondence relationship between the first index value and the second index value.

3. The information processing apparatus according to claim 1,
wherein the determination unit further determines whether or not noise is included in the acquired pulse wave waveforms of each of the parts based on the calculated first index value.

4. The information processing apparatus according to claim 3,
wherein the determination unit determines whether or not the acquired pulse wave waveform indicates the arrhythmia based on the calculated second index value.

5. The information processing apparatus according to any one of claims 1 to 4,
wherein the calculation unit calculates the first index value and the second index value in each period of a plurality of periods, and
wherein the determination unit determines whether or not the pulse wave waveform indicates the arrhythmia based on the first index value and the second index value which are calculated for each of the periods.

6. The information processing apparatus according to any one of claims 1 to 5,
wherein the information indicating the correspondence relationship between the first index value and the second index value is information indicating the correspondence relationship between the first index value and the second index value at least in one of a case where the pulse wave waveform is an arrhythmia and a case where the pulse wave waveform is not an arrhythmia, and based on the information indicating the correspondence relationship, the determination unit determines that the acquired pulse wave waveform indicates the arrhythmia in a case where it is determined that the calculated first index value is equal to or greater than the first index value that corresponds to the calculated second index value.

7. The information processing apparatus according to any one of claims 1 to 6,
wherein the calculation unit calculates a third index value indicating an intensity of correlation between the first index value and the second index value as information indicating a correspondence relationship between the first index value and the second index value, and
wherein the determination unit determines that the pulse wave waveform indicates the arrhythmia in a case where it is determined that the calculated third index value is small.

8. The information processing apparatus according to any one of claims 1 to 7,
wherein the determination unit calculates a ratio of a period during which it is determined that the second index value is smaller than a threshold value as information indicating the correspondence relationship between the first index value and the second index value and determines whether or not the pulse wave waveform indicates the arrhythmia based on the information.

9. The information processing apparatus according to any one of claims 1 to 8, further comprising:
a learning unit that generates information indicating the correspondence relationship between the first index value and the second index value based on the determination result on whether or not the determination unit indicates the arrhythmia.

10. The information processing apparatus according to any one of claims 1 to 9,
wherein the calculation unit calculates a fourth index value indicating a magnitude of variation of the second index value based on the second index value calculated for each period of the plurality of periods, and
wherein the determination unit switches information indicating the correspondence relationship between the first index value and the second index value according to the calculated fourth index value.

11. The information processing apparatus according to any one of claims 1 to 10,
wherein the second index value is a value indicating a matching degree between partial waveforms having a length that corresponds to a predetermined cycle in pulse wave waveforms of each of the parts.

12. The information processing apparatus according to any one of claims 1 to 11,
wherein the second index value is a value indicating a magnitude of variation of a pulse wave interval in the pulse wave waveforms of each of the parts.

13. An information processing method in which a computer executes processing of:

acquiring pulse wave waveforms of each of a plurality of parts obtained by analyzing images of each of the parts of a subject included in each photographed image obtained by photographing the subject;
calculating a first index value indicating a matching degree between the pulse wave waveforms of the acquired pulse wave waveforms of each of the parts;
calculating a second index value indicating a matching degree between partial waveforms of the acquired pulse wave waveforms of each of the parts;
determining whether or not the acquired pulse wave waveform indicates an arrhythmia based on a combination of the calculated first index value and the calculated second index value; and
outputting a determination result as to whether or not to indicate the arrhythmia.

14. An information processing program for causing a computer to execute processing of:

acquiring pulse wave waveforms of each of a plurality of parts obtained by analyzing images of each of the parts of a subject included in each photographed image obtained by photographing the subject;
calculating a first index value indicating a matching degree between the pulse wave waveforms of the acquired pulse wave waveforms of each of the parts;
calculating a second index value indicating a matching degree between partial waveforms of the acquired pulse wave waveforms of each of the parts;
determining whether or not the acquired pulse wave waveform indicates an arrhythmia based on a combination of the calculated first index value and the calculated second index value; and
outputting a determination result as to whether or not to indicate the arrhythmia.

# FIG. 1

MOVING IMAGE 101

(1-1)

WHEN SHAKING FACE

DEGREE OF CHANGE IN LIGHT AMOUNT VARIES ON LEFT CHEEK AND ON RIGHT CHEEK

CHEEK BECOMES SLIGHTLY BRIGHT

CHEEK BECOMES SLIGHTLY DARK

CALCULATE FIRST INDEX VALUE

(1-2)

CASE OF ARRHYTHMIA

PULSE WAVE WAVEFORM

NOT PERIODIC

CALCULATE SECOND INDEX VALUE

(1-3) DETERMINE WHETHER OR NOT TO INDICATE ARRHYTHMIA FROM FIRST INDEX VALUE AND SECOND INDEX VALUE

# FIG. 2

100

201 CPU

202 MEMORY

200

203 I/F

204 DISK DRIVE

206 CAMERA

210 NETWORK

205 DISK

FIG. 3

100

| 301 | 303 | 304 | 305 | 306 |
|-----|-----|-----|-----|-----|
| ACQUISITION UNIT | CALCULATION UNIT | DETERMINATION UNIT | ANALYSIS UNIT | OUTPUT UNIT |

# FIG. 4

# FIG. 5

# FIG. 6

# FIG. 7

...

# FIG. 8

FIG. 9

# FIG. 10

# FIG. 11

PULSE WAVE WAVEFORM
ON RIGHT SIDE OF FACE    1010

SHIFT WIDTH z

1011

1011

PULSE WAVE WAVEFORM
ON LEFT SIDE OF FACE    1020

1100

CALCULATE
MATCHING DEGREE

TIME t

LATEST TURNING POINT

FIG. 12

# FIG. 13

FIG. 14

FIG. 15

FIG. 16

# FIG. 17

# FIG. 18

START

S1801 — ACQUIRE MOVING IMAGE OBTAINED BY PHOTOGRAPHING SUBJECT WITHIN PREDETERMINED PERIOD

S1802 — EXECUTE GENERATION PROCESSING OF PULSE WAVE WAVEFORM FOR EACH OF PARTS

S1803 — HAS PULSE WAVE WAVEFORM BEEN ZERO-CROSSED? — NO

YES

S1804 — SET ONE OR PLURALITY OF ATTENTION PERIODS

S1805 — CALCULATE FIRST INDEX VALUE

S1806 — CALCULATE SECOND INDEX VALUE

S1807 — EXECUTE DETERMINATION PROCESSING BASED ON FIRST INDEX VALUE AND SECOND INDEX VALUE

# FIG. 19

START

S1901 — EXTRACT IMAGE

S1902 — ACQUIRE TIME SERIES DATA OF REPRESENTATIVE VALUE OF PREDETERMINED WAVELENGTH COMPONENT

S1903 — EXTRACT SIGNAL COMPONENT OF SPECIFIC FREQUENCY BAND FROM TIME SERIES DATA OF EACH OF WAVELENGTH COMPONENTS

S1907 — EXTRACT SIGNAL COMPONENT OF PULSE WAVE FREQUENCY BAND FROM TIME SERIES DATA OF EACH OF WAVELENGTH COMPONENTS

S1904 — EXTRACT TIME SERIES DATA OF ABSOLUTE VALUE OF SIGNAL COMPONENT OF SPECIFIC FREQUENCY BAND

S1905 — REMOVE STEEP FREQUENCY COMPONENT

S1906 — CALCULATE CORRECTION COEFFICIENT

S1908 — MULTIPLY CORRECTION COEFFICIENT

S1909 — SUBTRACT

S1910 — EXTRACT SIGNAL COMPONENT

END

# FIG. 20

START

S2001

IS FIRST INDEX VALUE EQUAL TO OR GREATER THAN FIRST THRESHOLD VALUE? — NO →

S2002

DETERMINE THAT NOISE IS INCLUDED IN PARTIAL WAVEFORM WITHIN ATTENTION PERIOD

YES ↓

S2003

IS SECOND INDEX VALUE EQUAL TO OR LESS THAN SECOND THRESHOLD VALUE? — NO →

YES ↓   S2005

DETERMINE THAT PARTIAL WAVEFORM WITHIN ATTENTION PERIOD INDICATES ARRHYTHMIA

S2004

DETERMINE THAT PARTIAL WAVEFORM WITHIN ATTENTION PERIOD INDICATES NORMAL PULSE

S2006

HAS PROCESSING PERFORMED WITH RESPECT TO ALL OF ATTENTION PERIODS?

NO

YES ↓

END

# FIG. 21

```
                    ( START )
                        │
                        ▼            ⟋ S2101
                  ╱──────────╲
                 ╱ IS FIRST    ╲
                ╱ INDEX VALUE    ╲        NO
               ⟨ EQUAL TO OR GREATER ⟩─────────────┐
                ╲ THAN FIRST THRESHOLD╱             │
                 ╲    VALUE?   ╱                    │
                  ╲──────────╱                      │
                        │ YES                       │
   S2103 ⟋              ▼                    S2102 ⟋▼
 ┌────────────────────────────┐      ┌────────────────────────────┐
 │ ACCUMULATE SECOND INDEX    │      │  DETERMINE WHEN NOISE IS   │
 │  VALUE AS STATISTICAL DATA │      │        INCLUDED            │
 └────────────────────────────┘      └────────────────────────────┘
                        │                            │
   S2104 ⟋              ▼◄───────────────────────────┘
                  ╱──────────╲
            NO   ╱ HAS PROCESSING╲
         ◄──────⟨ PERFORMED WITH RESPECT ⟩
                 ╲ TO ALL OF ATTENTION ╱
                  ╲   PERIODS?  ╱
                   ╲──────────╱
                        │ YES
   S2105 ⟋              ▼
                  ╱──────────╲
                 ╱ ARE THERE 60 ╲        NO
                ⟨ OR MORE PIECES OF STATISTICAL ⟩────────────┐
                 ╲    DATA?    ╱                             │
                  ╲──────────╱                               │
                        │ YES                                │
   S2106 ⟋              ▼                                    │
 ┌────────────────────────────┐                             │
 │ CALCULATE NUMBER OF SECOND │                             │
 │ INDEX VALUE WHICH IS EQUAL │                             │
 │ TO OR LESS THAN SECOND     │                             │
 │ THRESHOLD VALUE            │                             │
 └────────────────────────────┘                             │
                        │                                   │
   S2107 ⟋              ▼                                    │
                  ╱──────────╲          NO                   │
                 ⟨ IS RATIO GREATER ⟩─────────────┐          │
                  ╲  THAN 0.05?  ╱                │          │
                   ╲──────────╱                   │          │
                        │ YES                     │          │
   S2109 ⟋              ▼                  S2108 ⟋▼◄─────────┘
 ┌────────────────────────────┐      ┌────────────────────────────┐
 │ DETERMINE THAT PULSE WAVE  │      │ DETERMINE THAT PULSE WAVE  │
 │ WAVEFORM WITHIN PREDETERMINED│    │ WAVEFORM WITHIN PREDETERMINED│
 │ PERIOD INDICATES ARRHYTHMIA│      │ PERIOD INDICATES NORMAL PULSE│
 └────────────────────────────┘      └────────────────────────────┘
                        │                            │
                        ▼◄───────────────────────────┘
                    ( END )
```

EP 3 378 384 A1

# FIG. 22

START

S2201 — ACCUMULATE COMBINATION OF
FIRST INDEX VALUE AND SECOND
INDEX VALUE AS STATISTICAL DATA

S2202 — ARE THERE 60
OR MORE PIECES OF STATISTICAL
DATA? — NO

YES

S2203 — CALCULATE THIRD INDEX VALUE

S2204 — IS THIRD INDEX
VALUE EQUAL TO OR LESS THAN
THIRD THRESHOLD
VALUE? — NO

YES

S2206 — DETERMINE THAT PULSE WAVE
WAVEFORM WITHIN PREDETERMINED
PERIOD INDICATES ARRHYTHMIA

S2205 — DETERMINE THAT PULSE WAVE
WAVEFORM WITHIN PREDETERMINED
PERIOD INDICATES NORMAL PULSE

END

55

# FIG. 23

START

S2301
IS COMBINATION OF FIRST INDEX VALUE AND SECOND INDEX VALUE INCLUDED IN REGION?

YES → S2302
DETERMINE THAT PARTIAL WAVEFORM WITHIN ATTENTION PERIOD INDICATES ARRHYTHMIA

NO

S2303
IS FIRST INDEX VALUE EQUAL TO OR GREATER THAN FIRST THRESHOLD VALUE, OR IS SECOND INDEX VALUE EQUAL TO OR GREATER THAN SECOND THRESHOLD VALUE?

NO → S2304
DETERMINE THAT NOISE IS INCLUDED IN PARTIAL WAVEFORM WITHIN ATTENTION PERIOD

YES S2305
DETERMINE THAT PARTIAL WAVEFORM WITHIN ATTENTION PERIOD INDICATES NORMAL PULSE

S2306
HAS PROCESSING PERFORMED WITH RESPECT TO ALL OF ATTENTION PERIODS?

NO

YES

END

# FIG. 24A

START

S2401

IS MODEL SET? ——YES——

NO | S2403

EXECUTE DETERMINATION PROCESSING OF FIG. 20

S2404

ACCUMULATE COMBINATION OF FIRST INDEX VALUE AND SECOND INDEX VALUE AS STATISTICAL DATA

S2405

ARE THERE 60 OR MORE PIECES OF STATISTICAL DATA? ——NO——→

YES | S2406

CALCULATE NUMBER OF COMBINATIONS IN WHICH FIRST INDEX VALUE IS EQUAL TO OR GREATER THAN FIRST THRESHOLD VALUE AND SECOND INDEX VALUE IS EQUAL TO OR LESS THAN SECOND THRESHOLD VALUE

S2407

IS RATIO 0.00 OR EQUAL TO OR GREATER THAN 0.05? ——NO——

YES

( A )

S2402

EXECUTE DETERMINATION PROCESSING OF FIG. 23

S2408

INITIALIZE STATISTICAL DATA

( B )

# FIG. 24B

Ⓐ                                           Ⓑ

S2409

IS RATIO 0.00? — NO

YES ↓   S2410                             S2411

| ADOPT ACCUMULATED STATISTICAL DATA AS LEARNING DATA OF COMBINATION OF FIRST INDEX VALUE AND SECOND INDEX VALUE IN CASE OF NORMAL PULSE | ADOPT ACCUMULATED STATISTICAL DATA AS LEARNING DATA OF COMBINATION OF FIRST INDEX VALUE AND SECOND INDEX VALUE IN CASE OF ARRHYTHMIA |
|---|---|

S2412

INITIALIZE STATISTICAL DATA

S2413

IS THERE LEARNING DATA? — NO

YES ↓   S2414

GENERATE MODEL BASED ON LEARNING DATA

END

58

# FIG. 25A

START

S2501 — IS MODEL SET? — YES

S2503 — NO

EXECUTE DETERMINATION PROCESSING OF FIG. 20

S2502

EXECUTE DETERMINATION PROCESSING OF FIG. 23

S2504

ACCUMULATE COMBINATION OF FIRST INDEX VALUE AND SECOND INDEX VALUE AS STATISTICAL DATA

S2505 — ARE THERE 60 OR MORE PIECES OF STATISTICAL DATA? — NO

YES

S2506 — IS DIFFERENCE EQUAL TO OR GREATER THAN 0.4? — NO — C

YES

S2507 — CALCULATE THIRD INDEX VALUE

E

S2508 — IS THIRD INDEX VALUE EQUAL TO OR LESS THAN THIRD THRESHOLD VALUE? — YES

S2509 — NO

ADOPT ACCUMULATED STATISTICAL DATA AS LEARNING DATA OF COMBINATION OF FIRST INDEX VALUE AND SECOND INDEX VALUE IN CASE OF NORMAL PULSE

S2510

ADOPT ACCUMULATED STATISTICAL DATA AS LEARNING DATA OF COMBINATION OF FIRST INDEX VALUE AND SECOND INDEX VALUE IN CASE OF ARRHYTHMIA

D

S2517 — INITIALIZE STATISTICAL DATA

S2518 — IS THERE LEARNING DATA? — NO

S2519 — YES

GENERATE MODEL BASED ON LEARNING DATA

END

# FIG. 25B

C

S2511

CALCULATE NUMBER OF COMBINATIONS IN WHICH FIRST INDEX VALUE IS EQUAL TO OR GREATER THAN FIRST THRESHOLD VALUE AND SECOND INDEX VALUE IS EQUAL TO OR LESS THAN SECOND THRESHOLD VALUE

S2512

DETERMINE WHETHER RATIO IS 0.00 OR EQUAL TO OR GREATER THAN 0.05 — NO →

S2513

INITIALIZE STATISTICAL DATA

E

YES

S2514

IS RATIO 0.00? — NO →

S2516

ADOPT ACCUMULATED STATISTICAL DATA AS LEARNING DATA OF COMBINATION OF FIRST INDEX VALUE AND SECOND INDEX VALUE IN CASE OF ARRHYTHMIA

S2510 YES

S2515

ADOPT ACCUMULATED STATISTICAL DATA AS LEARNING DATA OF COMBINATION OF FIRST INDEX VALUE AND SECOND INDEX VALUE IN CASE OF NORMAL PULSE

D

## INTERNATIONAL SEARCH REPORT

| International application No. |
| --- |
| PCT/JP2015/082809 |

| A. CLASSIFICATION OF SUBJECT MATTER |
| --- |
| A61B5/02(2006.01)i |

According to International Patent Classification (IPC) or to both national classification and IPC

| B. FIELDS SEARCHED |
| --- |

Minimum documentation searched (classification system followed by classification symbols)
A61B5/00-5/03

| Documentation searched other than minimum documentation to the extent that such documents are included in the fields searched |
| --- |
| Jitsuyo Shinan Koho          1922–1996    Jitsuyo Shinan Toroku Koho    1996–2016 |
| Kokai Jitsuyo Shinan Koho    1971–2016    Toroku Jitsuyo Shinan Koho    1994–2016 |

Electronic data base consulted during the international search (name of data base and, where practicable, search terms used)
JSTPlus/JMEDPlus/JST7580(JDreamIII) & keyword: MYAKUHA, FUSEIMYAKU, GAZO, HAKEI, ICCHI, RUIJI, KINJI, SOKAN(in Japanese),

| C. DOCUMENTS CONSIDERED TO BE RELEVANT |
| --- |

| Category* | Citation of document, with indication, where appropriate, of the relevant passages | Relevant to claim No. |
| --- | --- | --- |
| A | JP 2006-247221 A  (Omron Healthcare Co., Ltd.), 21 September 2006 (21.09.2006), paragraphs [0007] to [0009], [0036] (Family: none) | 1–14 |
| A | JP 2004-521660 A  (Tensys Medical Inc.), 22 July 2004 (22.07.2004), paragraphs [0003], [0108] & US 2002/0062086 A1 paragraphs [0004], [0163] & US 2005/0038346 A1    & US 2006/0206032 A1 & WO 2001/070303 A2    & EP 1324788 A2 & AU 4766501 A          & CA 2403728 A1 | 1–14 |

| ☒ Further documents are listed in the continuation of Box C. | ☐ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 20 January 2016 (20.01.16) | 02 February 2016 (02.02.16) |

| Name and mailing address of the ISA/ | Authorized officer |
| --- | --- |
| Japan Patent Office 3-4-3,Kasumigaseki,Chiyoda-ku, Tokyo 100-8915,Japan | Telephone No. |

Form PCT/ISA/210 (second sheet) (January 2015)

**INTERNATIONAL SEARCH REPORT**

International application No.

PCT/JP2015/082809

C (Continuation). DOCUMENTS CONSIDERED TO BE RELEVANT

| Category* | Citation of document, with indication, where appropriate, of the relevant passages | Relevant to claim No. |
|---|---|---|
| A | WO 2015/121949 A1 (Fujitsu Ltd.), 20 August 2015 (20.08.2015), paragraphs [0009], [0049], [0073] (Family: none) | 1-14 |

Form PCT/ISA/210 (continuation of second sheet) (January 2015)

**INTERNATIONAL SEARCH REPORT**

| International application No. |
| --- |
| PCT/JP2015/082809 |

Continuation of B. FIELDS SEARCHED
Electronic data base consulted during the international search
(name of data base and, where practicable, search terms used)

PubMed & keyword: arrythmia, image, pulse wave

Form PCT/ISA/210 (extra sheet) (January 2015)

**EP 3 378 384 A1**

**REFERENCES CITED IN THE DESCRIPTION**

**Patent documents cited in the description**

- JP 2015082809 W
- JP 2000107147 A
- JP 2014502187 A
- JP 2014527863 A
- WO 2014038077 A
- JP 2014176584 A